# EUROPEAN PATENT APPLICATION

(11) **EP 3 017 840 A1**
(43) Date of publication of application: **11.05.2016**
(21) Application number: 15743844.1
(22) Date of filing: 09.01.2015
(51) Int. Cl.: A61M 25/00, A61M 25/095, G01B 15/00

(54) **LOCATION CONTROL SYSTEM**

(30) Priority: 01.02.2014 JP 2014018105
(71) Applicant: Sato, Hiroshi, Muroran-shi, Hokkaido 051-0013 (JP)
(72) Inventor: Sato, Hiroshi, Muroran-shi, Hokkaido 051-0013 (JP)
(74) Representative: LS-MP von Puttkamer Berngruber Loth Spuhler
(86) International application number: PCT/JP2015/050452
(87) International publication number: WO 2015/115152

(57) **Abstract**

The present invention provides a position control system which can constitute an injection-suction system capable of injecting liquid such as anti-cancer agents to a target position of a body and sucking a liquid such as cytosol from a target position of a body without destruction as possible. The position control system includes a movement apparatus 40 which can move a fine head 20 by magnetic force, and a position detection apparatus 50 which can detect a position of the fine head 20.

## Description

### TECHNICAL FIELD

The embodiment of the present invention relates to a position control system that can constitute an injection-suction system for injecting a liquid such as drug to a target position or sucking liquid such as cytoplasmic substrates from a target position in a human or animal body.

### BACKGROUND ART

In general, when delivering a drug inside tumor, a catheter is inserted into a vein, and an agent is injected into the vein from the catheter. However, it is difficult to spread the drug in tumor of few blood vessels, and increase a result of anti-cancer agents.

Thus, a catheter which have a precursor having a tubular shape and an incision member for incising a living tissue being mounted to the precursor has been proposed (for example, see PATENT LITERATURE 1.)

### CITATION LIST

### PATENT LITERATURE

PATENT LITERATURE 1:JP-A-2012-20105

### SUMMARY OF INVENTION

### TECHNICAL PROBLEM

However, there is a risk of destroying cells up to a tumor because an outer diameter of a conventional catheter is about 1 milimeter.

An object of the embodiment of the present invention is to provide a position control system which can constitute an injection-suction system capable of injecting liquid such as anti-cancer agents to a target position of a body and sucking a liquid such as cytosol from a target position of a body without destruction as possible.

### SOLUTION TO PROBLEM

For achieving the above object, one aspect of the present invention provide a position control system comprising:
a movement apparatus including an electromagnet capable of applying a magnetic field to a head that is formed from a magnetic material and can be moved through a body by the magnetic field, and a movement control unit capable of controlling a magnitude and an orientation of a magnetic force that acts on the head in response to the magnetic field applied by the electromagnet; and
a position detection apparatus being capable of determining a position of a mark existing on the head or in a tip end side position of a pipe which is attached to the head with a tip end thereof open and through which a liquid can be injected or suctioned via an opening portion in the tip end, the position detection apparatus being capable of detecting a position of the head on the basis of the position of the mark,
the movement control unit of the movement apparatus is capable of controlling an advancement direction of the head by adjusting the magnitude and the orientation of the magnetic force that acts on the head in response to the magnetic field applied by the electromagnet so that the pipe is inserted substantially linearly into a target position.

### ADVANTAGEOUS EFFECTS OF INVENTION

According to the embodiment of the present invention, an injection-suction system capable of injecting liquid such as anti-cancer agents to a target position of a body and sucking a liquid such as cytosol from a target position of a body without destruction as possible can be constituted.

### BRIEF DESCRIPTION OF THE DRAWINGS

Figure 1 is a perspective view showing a configuration example of a schematic of an injection-suction system according to the EXAMPLE 1 of the embodiment of the present invention.
Figure 2 is a partial perspective view of an injection-suction apparatus according to the EXAMPLE 1 of the embodiment of the present invention.
Figure 3A is a fragmentary plan view of an injection-suction apparatus according to the EXAMPLE 1 of the embodiment of the present invention, Figure 3B is an A-A line cross-sectional view of Figure 3A.
Figures 4A ∼ 4C are cross-sectional views illustrating an example of a production process of a ultra-fine pipe according to the EXAMPLE 1 of the embodiment of the present invention.
Figure 5 shows an example of a production process of an injection-suction apparatus according to the EXAMPLE 1 of the embodiment of the present invention, Figure 5A is a cross-sectional view in a plane direction, FIG. 5B is a lateral cross-sectional view. Figures 6A and 6B are conceptual views for explaining a method of inactivating a group of cancer cells together.
Figures 7A ∼ 7C are conceptual diagrams for explaining a method of inactivating plural groups of cancer cells together.
Figure 8 is a partial perspective view of an injection-suction apparatus according to the EXAMPLE 2 of the embodiment of the present invention.
Figure 9 shows an injection-suction apparatus according to the EXAMPLE 3 of the embodiment of the present invention,
Figure 9A is a partial perspective view, Figure 9B is a front view.
Figure 10 is a perspective view showing a configuration example of a schematic of an injection-suction system according to the EXAMPLE 4 of the embodiment of the present invention.
Figure 11 is a perspective view showing a configuration example of a schematic of an injection-suction system according to the EXAMPLE 5 of the embodiment of the present invention.
Figure 12 is a perspective view showing a configuration example of a schematic of an injection-suction system according to the EXAMPLE 6 of the embodiment of the present invention.
Figure 13 is a perspective view showing a configuration example of a schematic of an injection-suction system according to the EXAMPLE 7 of the embodiment of the present invention.
Figure 14 is a perspective view showing a configuration example of a schematic of an injection-suction system according to the EXAMPLE 8 of the embodiment of the present invention.
Figure 15 is a perspective view showing a configuration example of a schematic of an injection-suction system according to the EXAMPLE 9 of the embodiment of the present invention.
Figure 16 is a perspective view showing a configuration example of a schematic of an injection-suction system according to the EXAMPLE 10 of the embodiment of the present invention.
Figure 17 is a conceptual diagram for explaining an example of a method of position control of a fine head.
Figures 18A ∼ 18D are cross-sectional views showing an example of a position of a mark according to the EXAMPLE 1 of the embodiment of the present invention.
Figure 19 is a conceptual diagram for explaining an example of a procedure for detecting a position of a fine head.
Figure 20 is a conceptual diagram for explaining an example of a procedure for detecting a position of a fine head.
Figure 21 is a conceptual diagram for explaining an example of a procedure for detecting a position of a fine head.
Figure 22 is a conceptual diagram for explaining an example of a procedure for detecting a position of a fine head.

### DESCRIPTION OF EMBODIMENTS

The embodiments of the present invention are described below with reference to the drawings. In the drawings, for some of components having substantially same functions, a redundant description is omitted by denoting same reference sign.

### EXAMPLE 1

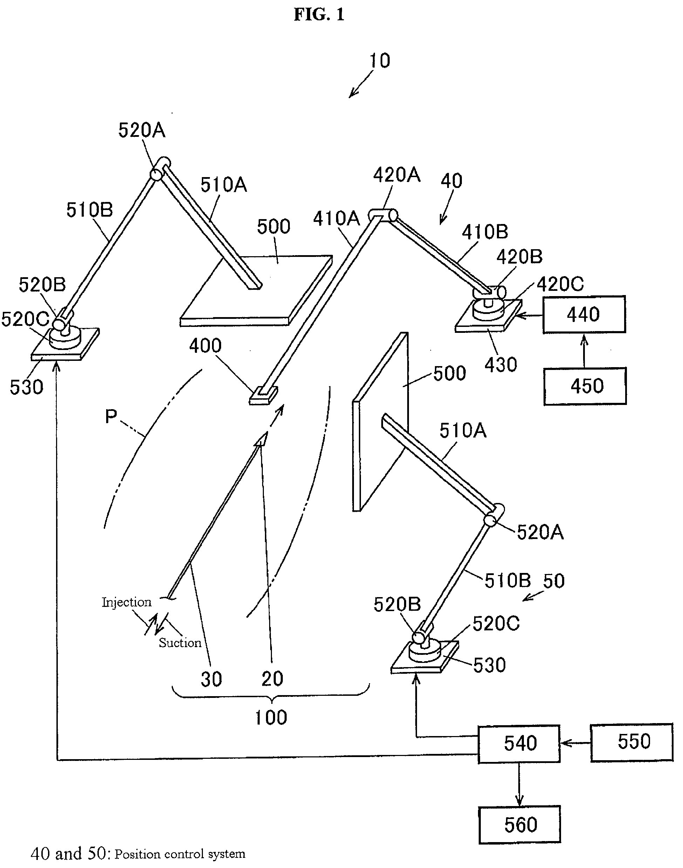

Figure 1 is a perspective view showing a configuration example of a schematic of an injection-suction system according to the EXAMPLE 1 of the embodiment of the present invention. Injection-suction system 10 is obtained by using the position control system of the embodiment of the present invention. Movement apparatus 40 and position detection apparatus 50 are an example of the position control system of the embodiment of the present invention. The injection-suction system 10 includes fine head 20 which can be moved by a magnetic field in a body such as a human body P, an injection-suction apparatus 100 having ultra-fine pipe 30 which is attached to the fine head 20 and can inject or suck a liquid, and the movement apparatus 40 which can move the fine head 20 by magnetic force, and the position detection apparatus 50 which can detect a position of the fine head 20.

### (Injection-suction apparatus)

Considering cell size (1 ∼ 100µm), a maximum width of the fine head 20 is preferably no greater than 100µm, more preferably no greater than 5µm so that cells are not destroyed upon insertion of the fine head 20 on a body as much as possible. In this embodiment, a maximum width of the fine head 20 is 1µm. Moreover, the fine head 20 is formed from a material having high permeability such as a magnetic material, for example, permalloy, silicon steel or the like. For more information about a structure of the fine head 20, it will be described later.

Ultra-fine pipe 30 is attached to the fine head 20 in a state in which a tip end is opened, liquid such as an anti-cancer agent is injected or liquid such as cytosol is sucked through an opening portion in the tip end. The ultra-fine pipe 30 has, for example, an outer diameter of 100nm ∼ 1µm and a length of 5cm ∼ 10m. The ultra-fine pipe 30 is formed from a material having low permeability such as non-magnetic material, for example, aluminum, silver, gold, quartz glass or the like. However, gold is usually not suitable for a material of a film 2000, which will be described later. In this embodiment, the ultra-fine pipe 30 has an inner diameter of 150nm, an outer diameter of 350nm, a length of a size of more than half of the diameter of much human body such as 50cm.

A pump which is formed by using, for example, an electric motor or a piezoelectric element is connected to the ultra-fine pipe 30. In addition, an injection-suction apparatus 100 may has just the fine head 20 and the ultra-fine pipe 30 without connecting the pump. In this case, that may be an injection-suction apparatus which can inject a drug by a weight of the drug in a manner of infusion, suck urine by inserting into a bladder of which a pressure is high, or the like.

### (Movement apparatus)

The movement apparatus 40 includes an electromagnet 400, first and second arms 410A and 410B for supporting the electromagnets 400, first and second rotary drive units 420A and 420B capable of rotating movement of the first arm 410A and the second arm 410B respectively around a horizontal axis, a third rotary drive unit 420C capable of rotating movement of the second rotary drive unit 420B around a vertical axis, a base 430 which supports the third rotary drive unit 420C, a control unit 440 capable of controlling each part of the movement apparatus 40, an operation unit 450 that can indicate a three-dimensional position to which the fine head 20 moves to the control unit 440. The first arm 410A and the second arm 410B, the first and second rotary drive units 420A and 420B, the third rotary drive unit 420C, the base 430, the control unit 440, the operation unit 450, are an example of a movement control unit of the movement apparatus being capable of controlling a magnitude and an orientation of a magnetic force that acts on the fine head in response to a magnetic field applied by the electromagnet.

The electromagnet 400 generates a magnetic field of which magnitude is in accordance with a magnitude of a current conducted. The electromagnet 400 may be a superconducting magnet.

The first to third rotary drive units 420A ∼ 420C, for example, can be configured using a motor having a movement part or a piezoelectric element or the like. The motor may be such as an electric motor. The first to third rotary drive units 420A ∼ 420C are connected to the first arm 410A and the second arm 410B, and move the electromagnet 400 by moving the first arm 410A and the second arm 410B. The first to third rotary drive units 420A ∼ 420C are an example of a drive unit with the movement control unit of the movement apparatus.

The operation unit 450 is configured to indicate a three-dimensional position to which the fine head 20 moves by, for example, lever operation or computer.

The control unit 440 moves the fine head 20 in a direction of indicated position i.e. target position based on a three-dimensional position indicated by the operation unit 450, by controlling a magnitude and an orientation of a magnetic force that acts on the fine head 20 by finely adjusting a strength of a magnetic field generated by the electromagnet 400 by controlling a magnitude of a current conducted to the electromagnet 400; and finely adjusting a position and an orientation of the electromagnet 400 by controlling the first to third rotation drive units 420A ∼ 420C.

For example, by performing control of an advancement direction of the fine head 20 to adjust a magnitude and a position and an orientation of the magnetic field applied to the fine head 20 by the electromagnet 400, that is, to adjust a magnitude and an orientation of the magnetic force that acts on the fine head 20 so as to suppress a bending of a movement path of the ultra-fine pipe 30, that is, as the ultra-fine pipe 30 is inserted substantially linearly into a target position, the ultra-fine pipe 30 does not get tangled, and a body tissue is not damaged so much when the fine head 20 and the ultra-fine pipe 30 are inserted into a body or are pulled out from a body. That is, problems such as the ultra-fine pipe 30 knots and the knot damages a body tissue when to move, and an internal circumference side of a curve increases power and a body tissue is cut if it is pulled to one direction in spite of orbit bending when the ultra-fine pipe 30 is very fine and the pipe in itself is sharp like a thread-like knife, are hard to occur. In this case, also advantage that it becomes easier to let the fine head 20 reach to a target position quickly because a movement distance up to the target position of the fine head 20 is shortened is obtained. Incidentally, the state is also included in a state that the ultra-fine pipe 30 has been inserted substantially linearly into a target position, to the extent that ultra-fine pipe 30 can not damage a body tissue so much for the reasons mentioned above, even if there is fold or bent or the like on a movement path of the ultra-fine pipe 30.

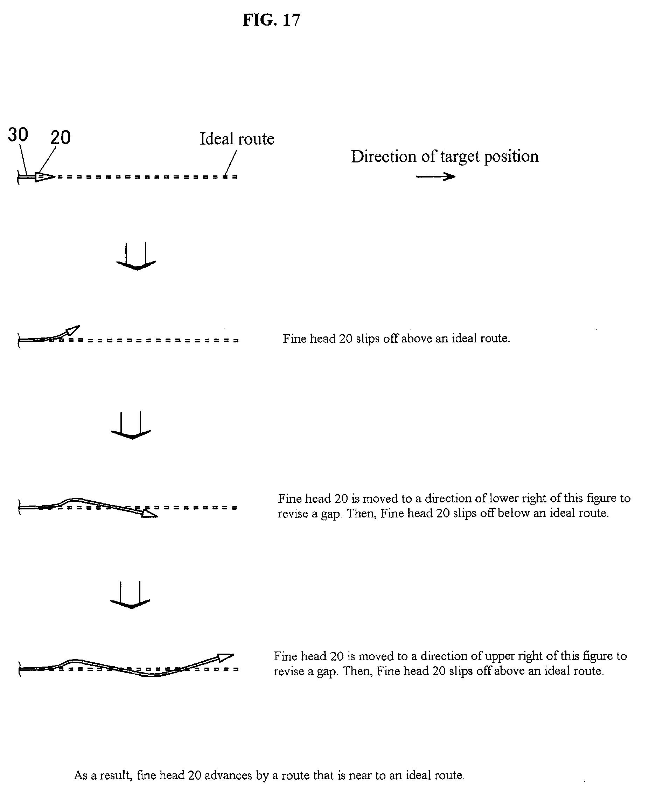

Position control of the fine head 20 by the control unit 440, for example, may be a simply control like that the fine head 20 is pulled in a direction of a target position, or a control like that it goes back to left if it goes to right too much, it goes back to right if it goes to left too much, it goes back to below if it goes to above too much, it goes back to above if it goes to below too much (see Figure 17). In particular, when of a control such as 'it goes back to left if it goes to right too much, it goes back to right if it goes to left too much, it goes back to below if it goes to above too much, it goes back to above if it goes to below too much', the fine head 20 becomes easier to reach a target position accurately even in a body of a human or animal having a complicated structure because when there is a deviation, the deviation can be corrected naturally.

The movement apparatus 40 may be provided with a rotary drive unit also in between the first arm 410A and the electromagnet 400.

In addition, the movement apparatus 40 may be one that the first arm 410A and the first rotary drive unit 420A are omitted and the second arm 410B is an elastic arm.

Moreover, the fine head 20 and the ultra-fine pipe 30 may be put in a container filled with water prior to use and the container with them may be adhered to a patient's body in use, a tip end of the ultra-fine pipe 30 may be moved by the movement apparatus 40 from there.

In the embodiment of the present invention, a purpose of using the movement apparatus 40 is to move a fine head.

### (Position detection apparatus)

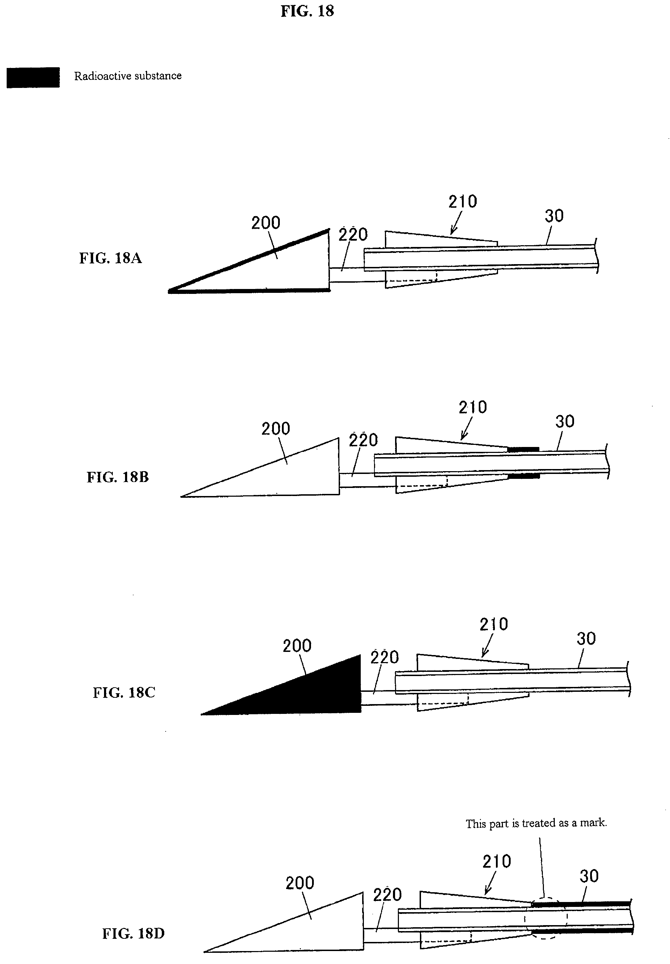

Position detection apparatus 50 includes a pair of X-ray CCD sensors 500 capable of detection and photographing radiation emitted from a mark of a radioactive substance which is applied to the fine head 20 (see Figure 18A), first and second arms 510A and 510B for supporting the X-ray CCD sensors 500, first and second rotary drive units 520A and 520B capable of rotating movement of the first arms 510A and the second arms 510B respectively around a horizontal axis, third rotary drive units 520C capable of rotating movement of the second rotary drive units 520B around a vertical axis, bases 530 which support the third rotary drive units 520C, a control unit 540 capable of driving and controlling each of rotary drive units 520A, 520B, 520C and detecting a position of the fine head 20, an operation unit 550 that can indicate three-dimensional positions of the X-rays CCD sensor 500, a display unit 560 that can display detected position of the fine head 20. A position of forming the mark of a radioactive substance may be also in a tip end side position of the ultra-fine pipe 30 (see Figure 18B). The display unit 560 may be omitted if a computer automatically peforms confirming that the fine head 20 has reached a target position. The X-ray CCD sensors 500 are an example of a radiographic imaging sensor which an imaging unit of the position detection apparatus has.

By detecting positions of the mark in a direction perpendicular to each other by the pair of X-ray CCD sensors 500, a three-dimensional position of the fine head 20 can be known.

The operation unit 550 is configured to indicate three-dimensional positions to which the X-ray CCD sensors 500 move by, for example, lever operation or computer.

The position detection apparatus 50 is configured so as to measure three-dimensional positions of tips of the first arms 510A, that is, three-dimensional positions of the X-ray CCD sensors 500 precisely (eg, in micrometer units) by providing position sensors, angle sensors or the like on joint portions between the first arms 510A and the second arms 510B, and joint portions between the first arms 510B and the bases 530, or providing three-dimensional position sensors at tips of the first arms 510A. The position sensors, the angle sensors, and the like provided on the joint portions, and the three-dimensional position sensors provided at the tips of the first arms 510A, are an example of a position detection unit for a radiographic imaging sensor capable of measuring a three-dimensional position of the radiographic imaging sensor with an imaging unit of the position detection apparatus.

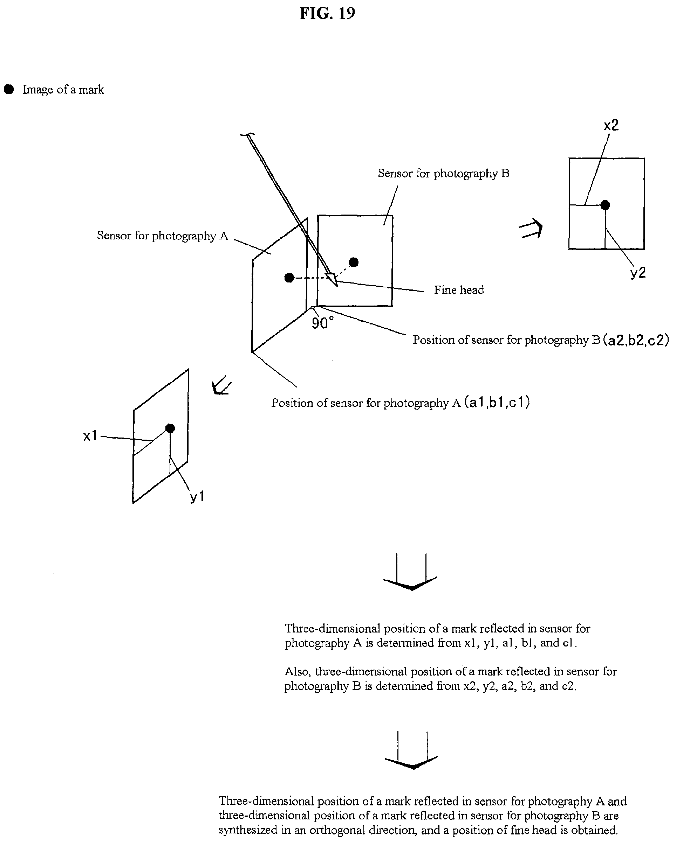

The control unit 540 is an example of a position detection unit. The control unit 540 includes an image recognition processing unit capable of performing image recognition processing that can recognize images by a pattern recognition. The control unit 540, for example, detects a position of the fine head 20 in the following procedure (see Figure 19):
1. Preparing a pair of image data photographed from a direction perpendicular to each other using the pair of X-ray CCD sensors 500;
2. In each of the image data, determining a two-dimensional position of a mark within an imaging range of the image data by searching the image data for a portion corresponding to the mark using the image recognition processing unit; and
3. Determining a three-dimensional position of the mark in the imaging range of each image data by adding a three-dimensional position of the X-ray CCD sensor 500 to a two-dimensional position of the mark in the imaging range of each image data, and synthesizing three-dimensional positions of the mark in the imaging range of a pair of image data in an orthogonal direction, and obtaining a position of the fine head 20.

Also, the control unit 540, based on three-dimensional positions indicated by the operation unit 550, by controlling the first to third rotation drive units 520A ∼ 520C, moves the X-ray CCD sensors 500 to indicated three-dimensional positions.

Incidentally, the mark is better to be in a tip end side position of the fine head 20 or the ultra-fine pipe 30, and the mark need not be formed separately from the fine head 20 and the ultra-fine pipe 30. In other words, it may be possible that a material of the fine head 20 is a substance capable of transmitting photographed by the X-ray CCD sensors 500, and the fine head 20 itself is treated as the mark (see Figure 18C). For example, a radioactive substance may be kneaded to the material of the fine head 20. In addition, it may be possible that a material of the ultra-fine pipe 30 is a substance capable of transmitting photographed by the X-ray CCD sensors 500 (see Figure 18D), and a total image or a wide range of image including a tip end side of the ultra-fine pipe 30 is projected in a photographed image data in the X-ray CCD sensors 500, and a portion corresponding to the tip end side in a image of the ultra-fine pipe 30 is treated as an image of the mark in the image recognition processing. For example, it may be possible that the ultra-fine pipe 30 is made kneading a radioactive substance to a material of the ultra-fine pipe 30.

A range which is marked by doctor hand or the like is in a range of accuracy of millimeters at most though it varies depending on a manual dexterity of the doctor corresponding to the work. In addition, the fine head 20 is just moved to 'generally' near center of the range in many cases. Therefore, it is possible to use large X-ray CCD sensors for digital X-ray imaging which are common that accuracy is low to reduce a cost because they are so huge as the position detection apparatus 50. It is also possible to fix the X-ray CCD sensors in a predetermined position without using the arms until a treatment is finished when using large X-ray CCD sensors for digital X-ray imaging as the position detection apparatus 50. A maintenance is facilitated in many cases because moving parts are reduced by eliminating the arms. If the fine head 20 is desired to move inside or outside of a cell membrane certainly, a movement of the fine head 20 with high accuracy is required. But an accuracy of a position detection may be also lower since it is possible to determine whether the tip end of the ultra-fine pipe 30 is inside or outside of a cell membrane by a method such as measuring a pressure by attaching a pressure gauge to the pump. Therefore, it is possible to use the large X-ray CCD sensors for digital X-ray imaging as the position detection apparatus 50 even in this case.

The position detection apparatus 50 may be provided with rotary drive units also in between the first arms 510A and the X-ray CCD sensors 500.

In addition, the position detection apparatus 50 may be one that the first arms 510A and the first rotary drive units 520A are omitted and the second arms 510B are elastic arms.

Moreover, it is also possible to use X-ray CMOS sensors in place of the X-ray CCD sensors.

In the embodiment of the present invention, a purpose of using the position detection apparatus 50 is to determine a position of a fine head or a tip end side of an ultra-fine pipe.

### (Detailed structure of fine head)

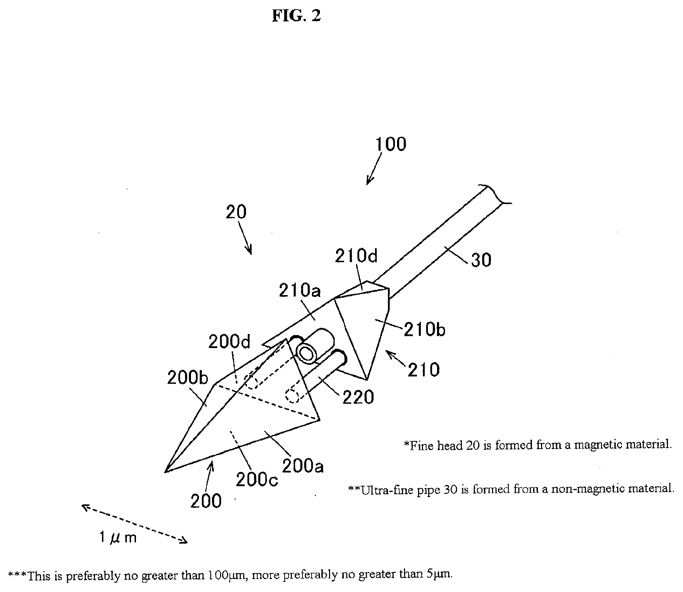

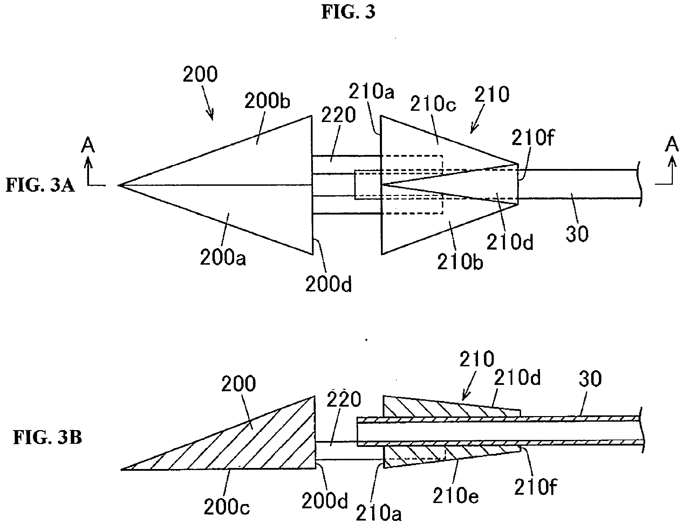

Figure 2 is a perspective view of the fine head 20 according to the EXAMPLE 1 of the embodiment of the present invention, Figure 3A is a plan view of the fine head 20 according to the EXAMPLE 1 of the embodiment of the present invention, Figure 3B is an A-A line cross-sectional view of Figure 3A.

The fine head 20 includes a first head portion 200 locates on a front side, a second head portion 210 locates on a rear side, a pair of connecting portions 220 that connects between the first head portion 200 and the second head portion 210, and has, for example, a rugby ball shape as a whole. Incidentally, an overall shape of the fine head 20 may be such as prismatic or cylindrical if it is fine-sharp.

The first head portion 200, for example, is shaped to taper toward a tip end thereof such as a substantially triangular pyramid, and provided with sides 200a, 200b, and 200c, a bottom surface 200d. The first head portion 200 is formed as it can make incisions in living tissue, that is, for example, a tip is formed at an acute angle. The first head portion 200, for example, is formed from a material having high magnetic permeability. In addition, lattice constants of the materials of the first head portion 200 and the connecting portions 220, may be a value as the first head portion 200 and the connecting portions 220 adhere to a liquid material for forming the second head portion 210. A part of the first head portion 200 may be formed from a different material to material of other parts. For example, a tip portion may be formed from a material having low magnetic permeability, and other parts may be formed from a material having high magnetic permeability. The first head portion 200 may have a protrusion such as a wire-shaped or blade-shaped as part.

The second head portion 210, for example, is shaped to taper toward a rear end thereof such as a substantially triangular pyramid of which vertex is flat, and provided with a bottom surface 210a, sides 210b, 210c, and 210e, a top surface 210f. The second head portion 210, for example, is formed from a material having high magnetic permeability. The second head portion 210 may be formed from a material of which a melting point is less than one of the first head portion 200 (eg, A low-melting point alloy). The first and second head portions 200 and 210 are formed from a same material. In addition, either the first head portion 200 or the second head portion 210 may be formed from a material having high magnetic permeability, and the other may be formed from a material having low magnetic permeability. A part of the second head portion 210 may be formed from a different material to material of other parts. For example, a rear portion may be formed from a material having low magnetic permeability, and other parts may be formed from a material having high magnetic permeability. The first head portion 200 may have a protrusion such as a wire-shaped or blade-shaped as part.

The connecting portions 220 are formed integrally with the first head portion 200 in this embodiment, but the connecting portions 220 may be formed separately and attached to the first head portion 200. The connecting portions 220 may be one piece or more than two pieces.

The ultra-fine pipe 30 is provided so that its tip is positioned between the first and second head portions 200 and 210. In addition, the tip of the ultra-fine pipe 30 may be positioned in other than between the first and second head portions 200 and 210.

### (Production method of injection-suction apparatus)

Then, a production method of the injection-suction apparatus 100 is described.

### (1) Production of first head portion and connecting portion of fine head

The first head portion 200 of the fine head 20 is produced by fine three-dimensional structure forming method using a focused ion beam (FIB) (eg, see JP-A-2004-291140). In other words, by blowing a raw material gas containing a material of the first head portion 200 from a gas nozzle to a substrate in advance and irradiating the FIB thereto, a fine three-dimensional structure formed from decomposition product of the raw material gas is deposited onto the substrate. Since a branch-like protrusion is formed on a side surface of the deposited fine three-dimensional structure, the protrusion is removed by irradiating the FIB. Then, the first head portion 200 is produced by removing the substrate from the fine three-dimensional structure.

Then, the pair of connecting portions 220 is formed by the fine three-dimensional structure forming method on the bottom surface 200d of the first head portion 200. Thus, the first head portion 200 having the connecting portions 220 is made.

Then, a mark formed from a radioactive substance is applied to the first head portion 200 or the second head portion 210 (see Figure 18A). In addition, a radioactive substance may be kneaded to a material of the first head portion 200 or the second head portion 210 (see Figure 18C).

### (2) Production of ultra-fine pipe

The ultra-fine pipe 30 is produced by such procedure as described below using such as photolithography similar to such as a fabrication process of an inkjet printer nozzle.

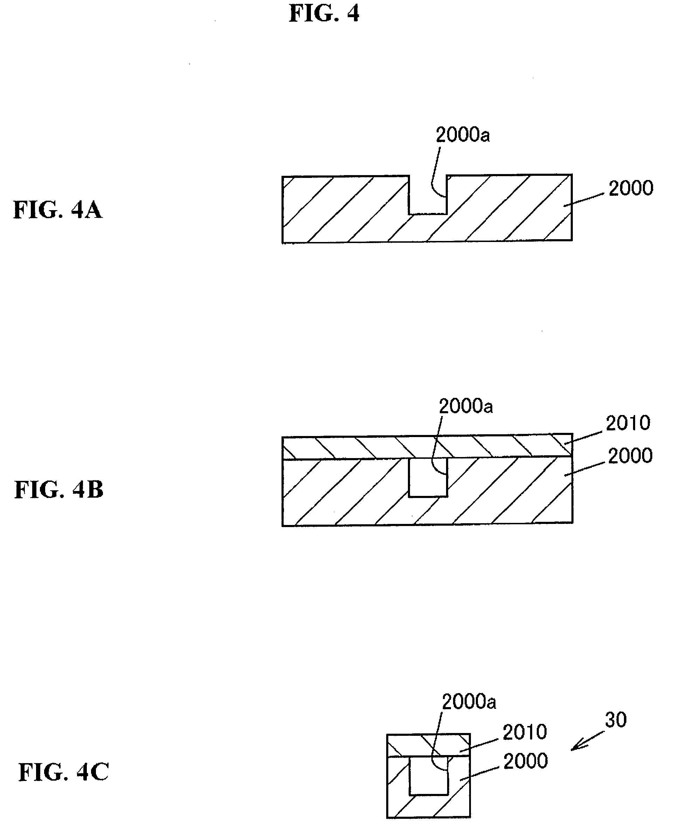

First, a thin (eg, 250nm thickness) film 2000 is formed by vapor deposition or the like, and a shallow thin (eg, depth of 150nm, width of 150nm) groove 2000a is formed on the film 2000 by photolithography (see Figure 4A).

Next, the groove 2000a is covered with a film 2010 which is thin (eg, 100nm thickness) and is formed from a material having lower melting point than the film 2000 (see Figure 4B). In this case, since molecules would penetrate also in the groove 2000a if the film 2010 is directly deposited by vapor deposition apparatus, the film 2010 which was formed on other substrate using vapor deposition apparatus (for example, a larger film which can cover the groove also when a place where the film falls into gets out of a position a little) may be arranged by a method such as to fall in a room of which a vacuum degree is high. Incidentally, it is preferable to select a material so that lattice constants of a materials of the film 2000 and the film 2010 become a value as the film 2000 and the film 2010 are integrated when the film 2000 is covered with the film 2010. Moreover, both of the film 2000 and film 2010 are preferably formed from a material having a low magnetic permeability.

Then, the film 2010 is melted by heating the film 2000 and the film 2010 to a temperature at which the film 2010 melts and the film 2000 does not melt. Thereafter, the film 2010 is cooled and hardened, and is integrated with the film 2000. The film 2010 may be melted and hardened by chemical reaction with added chemicals instead of heating and cooling. Incidentally, molten film 2010 does not flow into an inside of the groove 2000a as long as it is not pushed since a liquid has a surface tension.

Next, the film 2010 is cut in a thickness direction of the film 2010 leaving both sides of the groove 2000a 100nm respectively by, for example, a micro-processing machine such as a focused ion beam processing machine capable of performing a processing of 100nm width (see Figure 4C). In this way, the ultra-fine pipe 30 having an inner diameter of 150nm and an outer diameter of 350nm is produced.

The ultra-fine pipe 30 is better to be longer (eg, 50cm) when inserting the ultra-fine pipe 30 into a body, but a production of a photo mask for forming the groove 2000a (Pattern drawing, etc.) and the cutting of the film 2000 and 2010 can be performed in a short time since most of the pattern is straight, and so a productivity is relatively high.

### (3) Production of second portion of fine head and consolidation of fine head and ultra-fine pipe

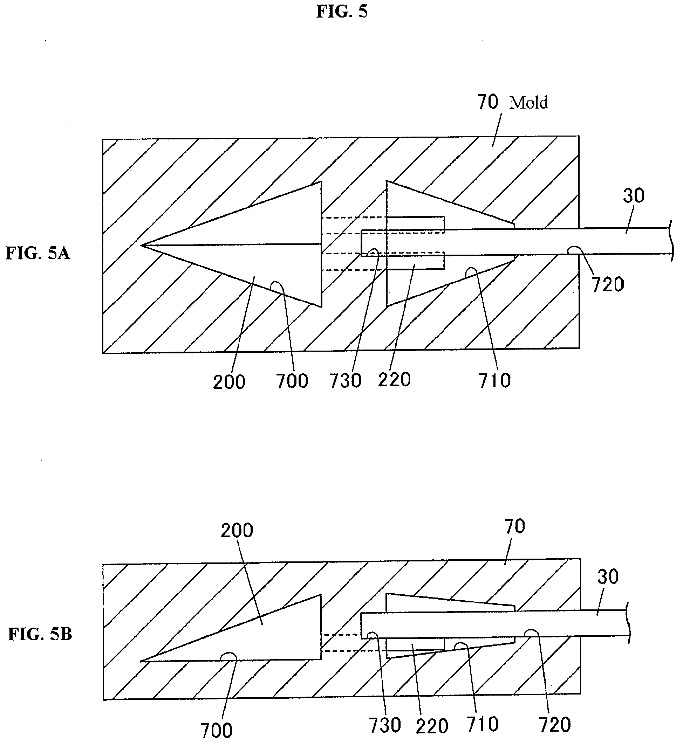

In this embodiment, a mold 70 shown in Figure 5 which is produced by fine processing such as photolithography or focused ion beam processing is used. The mold 70 has been divided into a plurality, and includes the first space portion 700 for accommodating the first head portion 200, the second space portion 61 for forming the second head portion 210, and a through-hole 720 and recesses 730 for accommodating the ultra-fine pipe 30. In addition, a lattice constant of a material of the mold 70 is a value as a material to be injected into the second space portion 61 does not adhere to the mold 70. Incidentally, the mold 70 need not to be divided. For example, one mold which upper surface of the mold is open and a flat surface out of surfaces constituting the second head portion 210 is correspond to the upper surface of the mold 70, may be used.

As shown in Figure 5, the first head portion 200 is accommodated in the first space portion 700 of the mold 70, the ultra-fine pipe 30 is accommodated into the through-hole 720 and the recesses 730.

Then, a liquid material is injected in the second space portion 701 from an injection hole (not shown) of the mold 70. A tip of the ultra-fine pipe 30 is not clogged with the liquid material because the tip of the ultra-fine pipe 30 is inserted into the recesses 730 of the mold 70. A clogging may be eliminated by cutting a tip of the ultra-fine pipes 30 after the liquid material has solidified instead of providing the recesses 730.

Then, the liquid material that was injected into the second space portion 61 is solidified. Solidifying the liquid material may be a cooling or a chemical reaction with added chemicals. Incidentally, a lattice constant of the liquid material after solidified is preferred to be a value which the liquid material after solidified adheres to the ultra-fine pipe 30 and the connecting portions 220, and does not adhere to the mold 70.

Finally, the injection-suction apparatus 100 is taken out from the mold 70 by separating the mold 70. As described above, the second head portion 210 is produced, then the injection-suction apparatus 100 which the ultra-fine pipe 30 is consolidated with the second head portion 210 is produced.

The second head portion 210 was produced using the mold 70 in the this embodiment, but the second head portion 210 may be produced using fine processing such as focused ion beam. In this case, the second head portion 210 may be produced in a way that, in advance a hole for a passage of the ultra-fine pipe 30 is made in the second head portion 210, the ultra-fine pipe 30 is picked up and fitted in the hole by a probe of a scanning probe microscope (SPM) or the like.

Furthermore, the head portions 200 and 210 need not to be divided. For example, the head portion may be formed integrally like the head portion of just a shape that a portion corresponding to the first head portion 200 is consolidated with a portion corresponding to the connecting portions 220 without a portion corresponding to the second head portion 210, or the head portion of a shape that has just one cylindrical or prismatic. In that case, a part of integrally formed head portion may be formed from a different material to material of other parts. For example, a tip portion may be formed from a material having low magnetic permeability, and other parts may be formed from a material having high magnetic permeability. Integrally formed head portion may have a protrusion such as a wire-shaped or blade-shaped as part.

The head portion may be divided into three or more. In that case, a part of the divided head portion may be formed from a material having high magnetic permeability, and other parts of the divided head portion may be formed from a material having low magnetic permeability. A part of each divided head portion may be formed from a different material to material of other parts. For example, in the divided head portion positioned on a tip end side, a tip end portion may be formed from a material having low magnetic permeability, and other parts may be formed from a material having high magnetic permeability. Each divided head portion may have a protrusion such as a wire-shaped or blade-shaped as part.

Moreover, whether the head portion is divided or not, the head portion may be adhered to the ultra-fine pipes 30 by using an adhesive. In this case, a width of the head portion may be smaller than an outer diameter of the ultra-fine pipe 30 if the tip of the ultra-fine pipe 30 is fine-sharp.

### (Operation of injection-suction system)

Next, an operation example of the injection-suction system 10 is described separately when (1) anti-cancer therapy and (2) brain tumor treatment.

### (1) anti-cancer therapy

First, a patient is photographed using X-ray CT apparatus, MRI (magnetic resonance imaging) apparatus, PET (positron emission tomography) apparatus or the like, and a three-dimensional image of an affected area is obtained. Furthermore, until the therapy is completed, it is better that a body is firmly fixed by using such as a metal fitting so as not to move as with general cancer radiation therapy.

Then, a doctor looks at the obtained three-dimensional image of the patient, and performs a three-dimensional markings for affected area, that is, a target position. The marking may be performed automatically by a computer. High-precision automatic detection can not be performed in a current image processing technology level, but it is sufficient even in automatic detection by a computer since marking by a hand of the doctor is the best in millimeters precision and it is enough if there is precision to follow that. For example, three-dimensional markings may be performed like that a marking on an image as viewed from a certain direction is performed, and a marking on an image as viewed from a direction perpendicular to it is performed, and the two marking positions are synthesized in a three-dimensional coordinate.

Next, the doctor operates the operation unit 450 of the movement apparatus 40, and indicates a three-dimensional position to which the fine head 20 of the injection-suction apparatus 100 moves, for example, a position near a center of a range of the marking. The control unit 440 inserts the fine head 20 into a body and moves the fine head 20 in a direction of target position, that is, indicated three-dimensional position based on a three-dimensional position indicated by the operation unit 450, by controlling a magnitude and an orientation of a magnetic force that acts on the fine head 20 by controlling the electromagnet 400 and the first to third rotation drive units 420A ∼ 420C. The operation by the doctor may be performed automatically by a computer instead of the doctor.

A movement of the X-ray CCD sensors 500 and scanning by X-ray CCD sensors 500 are repeated until a pixel which gives a certain value or more of radiation is in the screen is found. Once found, it is thereafter repeated scanning by moving the X-ray CCD sensors 500 as to track a movement of that pixel. Real-time scanning is possible, since it is less amount of calculation very much than an image forming calculations for such as CT because the pixel which gives a certain value or more of radiation is usually just one pixel in all of pixels in the CCD and just a process of searching for it is performed. Movement of the X-ray CCD sensors 500 and scanning by X-ray CCD sensors 500 may be performed automatically by a computer.

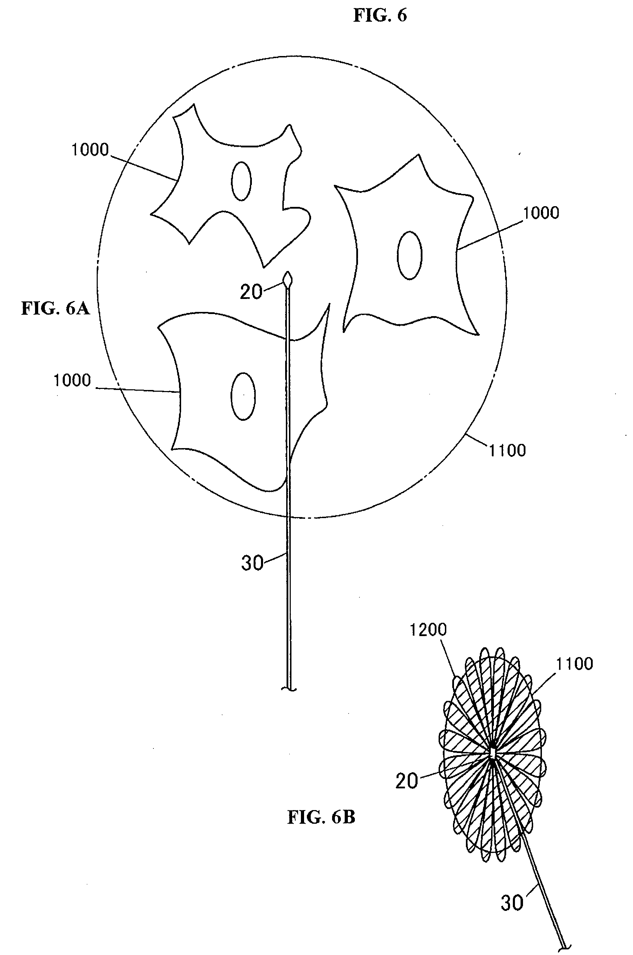

Next, the doctor checks a position of the fine head 20 by the position detection apparatus 50. When it is confirmed that the fine head 20 has reached a target position, an anti-cancer agent is injected into the target position via the ultra-fine pipe 30 by the pump. Plural cancer cells 1000 constitute a group of cancer cells 1100 (a mass of connected cells) as shown in Figure 6A, and the cancer cells 1000 in the group of cancer cells 1100 is inactivated by injecting anti-cancer agent 1200 in the center of the group of cancer cells 1100 as shown in Figure 6B. Confirmation that the fine head 20 has reached a target position may be performed automatically by a computer.

The fine head 20 is pulled out from a body by holding and linearly pulling a portion of the ultra-fine pipe 30 which extends outside the body after the injection of the anti-cancer agent is complete. When pulling the fine head 20 out, it may be possible that a base of the ultra-fine pipe 30 is disconnected and the ultra-fine pipe 30 is pulled to back by applying a magnetic field to the fine head 20 instead of pulling forward.

If a position of the cancer cells 1000 which are treated next is close to a treatment part immediately before, it may be possible that all the ultra-fine pipe 30 is not pulled out but just pulled slightly from a body side, and the fine head 20 is moved to the position of the cancer cells 1000 which are treated next from there.

If there are some marking ranges, the above-described operation is repeated for all rest of the marking ranges.

Incidentally, it may be possible that the above operation is repeated again at intervals of a predetermined time (for example, weeks to months) of which length is determined at depending on such as a division rate of a target cancer cells until the cancer cells 1000 disappear completely after inactivating the cancer cells 1000 once by the above operation.

Even when it is not possible to recognize a group of cancer cells from an obtained image by photographing a patient because the group of cancer cells are small, it is possible to inactivate substantially all cells of groups of cancer cells existing in a body of a patient even in current situation in which just relatively large cancer cells can be detected, by repeating to inactivate cancer cells before the cancer cells become terminal cancer, that is, the cancer cells erode with thick blood vessel as possible and after the cancer cells grow to a size capable of being recognized. A number of times of the inactivating is repeated varies depending on a state such as a division rate and ease of diffusion to a whole body of a target cancer cells.

Because a division rate of cancer cells is to the extent that a group of cancer cells become a double size in one month, that is, division of cancer cells is about once a month at the earliest; and the inactivation by not a cell unit but a group unit as described above can be performed in this embodiment, it is considered that a pace of inactivating cancer cells in this embodiment rarely does not keep up with a pace of division of cancer cells. Even if not keep up, there is an effect of delaying a progression of cancer.

When injecting the anti-cancer agent 1200, the fine head 20 may be moved to outside cells which locates near the three-dimensional position indicated by the operation unit 450, that is, an outside of cell membranes of the cells. It may be distinguished whether the fine head 20, that is, a tip of the ultra-fine pipe 30 locates inside the cell membrane or not by, for example, examining a magnitude of pressure applied to an interior of the ultra-fine pipe 30 by attaching a pressure gauge in a pump for injecting an anti-cancer agent through the ultra-fine pipe 30 utilizing condition of there existing a difference in pressure required for injection or suction between inside and outside of the cell membrane (due to such as a difference in viscosity of cytoplasmic substrates and interstitial fluid), and determining whether the magnitude of the pressure is the inside one or outside one. In that case, it may be possible that the pressure is re-measured while advancing or retreating the tip of the ultra-fine pipe 30 little by little, for example, by length roughly from a fraction of up to one-half of an average total length of the cells around the tip of the ultra-fine pipe 30 if the tip of the ultra-fine pipe 30 locates inside the cell membrane. If the tip of the ultra-fine pipe 30 locates outside of the cell membranes of the cells, the injected anti-cancer agent is easily diffused.

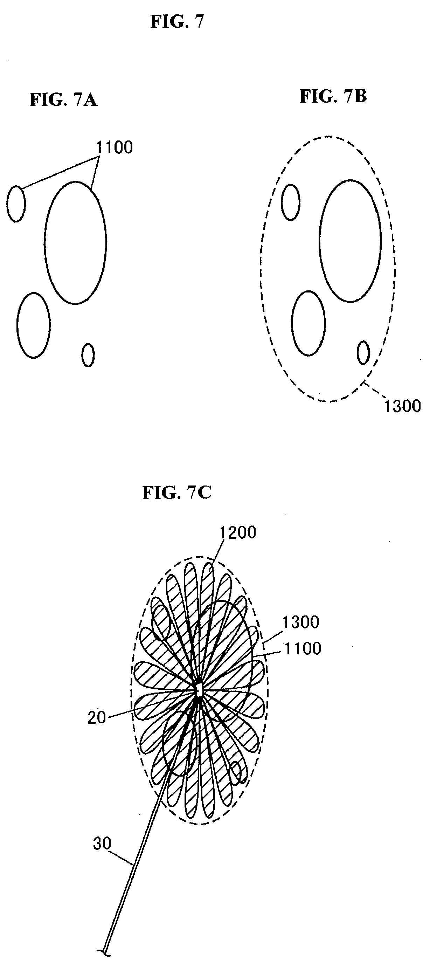

As shown in Figure 7A, plural groups of cancer cells 1100 which locates close to each other may be inactivated together as summarized one group of cancer cells 1300. In this case, it may be possible that the anti-cancer agent 1200 is injected into a center of the summarized one group of cancer cells 1300 and the anti-cancer agent 1200 is diffused from there. By inactivating plural groups of cancer cells 1100 together, it is possible to reduce a burden on a patient and speed up a process significantly.

An anti-cancer agent has been used as a drug in the above anti-cancer therapy, but drugs other than an anti-cancer agent may be used. In addition, it is preferable that a drug which corresponds to a target position is used. For example, if cancer cells of a target position is osteosarcoma, it may be used a drug such as hydrochloric acid capable of dissolving the osteosarcoma instead of an anti-cancer agent. In this case, it may be possible that the fine head 20 is advanced while dissolving a bone that exists on a path leading to the osteosarcoma by using the injection-suction apparatus 100. If a treatment object contains both osteosarcoma and cancer cells other than osteosarcoma, it may be possible that a couple of combinations of the fine head 20 and the ultra-fine pipe 30 and a pump are set up and selectively used for the osteosarcoma and the cancer cells other than osteosarcoma. A drug that alters a function of a gene in cancer cells of a target position.

### (2) brain tumor treatment

In this case, a treatment may be performed by such method as the above anti-cancer therapy, or the following procedure. First, a patient is photographed using X-ray CT apparatus, MRI (magnetic resonance imaging) apparatus, PET (positron emission tomography) apparatus or the like, and a three-dimensional image in which a brain tumor that is an affected area can be distinguished is obtained.

Then, a doctor looks at the obtained three-dimensional image of the patient, and performs a three-dimensional markings for a range including a range to be shrunk. In this case, it is preferable to perform the marking in consideration so as not to bore a large hole in a vessel wall of thick blood vessels. Because it is difficult to calculate the range to be shrunk from a CT image, a MRI image, a PET image or the like automatically and precisely, that may be performed by an eye of a doctor.

Then, the fine head 20 is moved to inside cell which locates in the marking range, that is, an inside of a cell membrane of the cell using the movement apparatus 40 as described above. It may be distinguished whether the fine head 20, that is, a tip of the ultra-fine pipe 30 locates inside the cell membrane or not by, for example, examining a magnitude of pressure applied to an interior of the ultra-fine pipe 30 by attaching a pressure gauge in a pump for injecting an anti-cancer agent through the ultra-fine pipe 30 utilizing condition of there existing a difference in pressure required for injection or suction between inside and outside of the cell membrane (due to such as a difference in viscosity of cytoplasmic substrates and interstitial fluid); and determining whether the magnitude of the pressure is the inside one or outside one. In that case, it may be possible that the pressure is re-measured while advancing or retreating the tip of the ultra-fine pipe 30 little by little, for example, by length roughly from a fraction of up to one-half of an average total length of the cells around the tip of the ultra-fine pipe 30 if the tip of the ultra-fine pipe 30 locates outside the cell membranes.

Then, cytoplasmic substrates is sucked out by means of a pump through ultra-fine pipe 30.

Then, the fine head 20 is pulled out from a body by holding and linearly pulling a portion of the ultra-fine pipe 30 which extends outside the body.

Then, cells in the marking range are shrunk while moving a tip of the ultra-fine pipe 30 little by little like groping for cells so that other cells in the marking range are also inactivated.

If a position of cells which are treated next is close to a treatment part immediately before, it may be possible that all the ultra-fine pipe 30 is not pulled out but just pulled slightly from a body side, and the fine head 20 is moved to the position of the cells which are treated next from there.

When it is concluded that cells to be shrunk in the range have been shrunk sufficiently, the treatment is complete.

In the case of the brain tumor treatment, it may be possible that, in advance holes are bored in a plurality of spaced locations on a skull by a drill or the like, the ultra-fine pipe 30 is inserted from a hole via which a tip of the ultra-fine pipe 30 can advance to a target position without being caught on the skull among those holes.

### (Effect of this embodiment)

According to this embodiment, the following effects.
(A) Invasiveness of a catheter can be lowered according to a narrowness of a diameter. However, a torque transmission performance of a guide wire enough to be pushed into a body by hand of man is difficult to be obtained because the guide wire becomes soft even if it is formed from a hard material when the guide wire has a very thin diameter (for example, 1 micrometer diameter, etc.). In addition, it is all the more difficult for a pipe because the pipe is a hollow, so it is difficult to have a catheter of which diameter is very thin enough sufficient torque transmission performance. For this reason, it was difficult to make a catheter of which diameter is very thin conventionally. However, an injection-suction similar to one of a catheter of which diameter is very thin (for example, I micrometer diameter, etc.) can be performed in this embodiment because it is possible to insert a pipe of which diameter is very thin into a body by the fine head 20 formed from a magnetic material being attached to a tip end of the ultra-fine pipe 30 formed from a nonmagnetic material, and moving the fine head 20 by a magnetic force.
(B) The fine head 20 has a structure divided into two front and rear portions, and an opening portion at a tip end of the ultra-fine pipe 30 is rarely clogged by such as a piece of a cell membrane if the tip end of the ultra-fine pipe 30 positions between the portions.
(C) In this embodiment, the fine head 20 and the ultra-fine pipe 30 can be moved to many positions by repeating this inserting and pulling, and a large number of cells or groups of cancer cells can be inactivated in a short time, because the fine head 20 does not destroy too much organs also when it goes back and forth in a body many times.
(D) It is possible that such as a high concentration of anti-cancer agent is injected into a single cell at a pin point and cytoplasmic substrates of a single cell is sucked to shrink the cell at a pin point because a very small amount of injection or suction can be performed by using a pump which makes use of an electric motor or a piezoelectric element for a injection or suction via the ultra-fine pipe 30.
(E) A calculation amount of an image forming calculation in such as CT and MRI is large generally, but an amount of calculation is very small and position detection can be performed at a high frame rate and high-speed response, that is, in real time in a position detecting process in this embodiment because it is just required that a pixel having a luminance value more than a reference value, that is, a position of a head that emits such as radiation which normally exists just around one is searched from among pixels constituting a certain image and a two-dimensional position of the fine head 20 measured by the X-ray CCD sensors 500 is added to a three-dimensional position of a tip end of arm.
(F) A bottom surface of a cone or pyramid is likely to get caught in a body when pulling out a fine head from the body if a shape of the fine head is conical or pyramidal, but the fine head 20 can be pulled out smoothly from a body when an overall shape of the fine head 20 is a rugby ball shape.
(G) The fine head 20 is easy to recognize even when it is very fine because the fine head 20 is projected larger than an original size in the X-ray CCD sensors 500 since a radioactive substance emits radiation radially. Incidentally, an anti-cancer therapy and a brain tumor treatment which were described in this embodiment can be used for not only human but animals such as pet.

### EXAMPLE 2

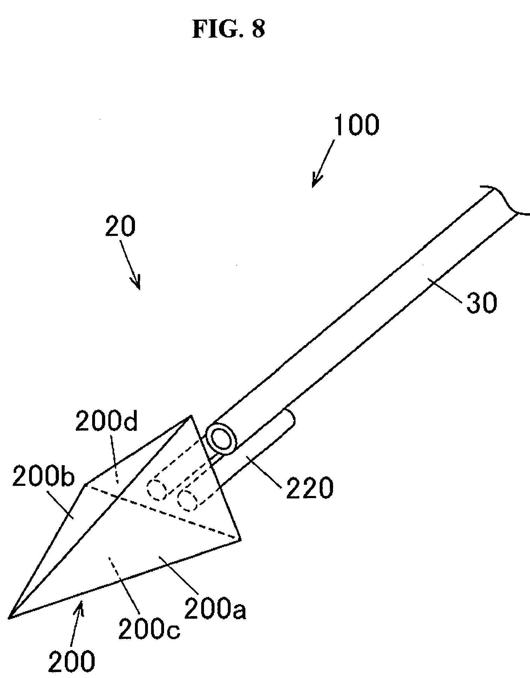

Figure 8 is a partial perspective view of an injection-suction apparatus according to the EXAMPLE 2 of the embodiment of the present invention. The first and second head portions 200 and 210 and the connecting portions 220 constitute the fine head 20 of the injection-suction apparatus 100 in the EXAMPLE 1, but the first head portion 200 and the connecting portions 220 constitute the fine head 20 in this embodiment.

The first head portion 200 and the connecting portions 220 in the fine head 20 according to the EXAMPLE 2 may be formed using such as focused ion beam (FIB). An interval of the pair of connecting portions 220 may be a distance in contact with the ultra-fine pipe 30. Incidentally, it may be possible that, in advance the first head portion 200 and the pair of connecting portions 220 prepared separately, the first head portion 200 is bonded to the pair of connecting portions 220. The connection portions 220 may be constituted by one part or three parts or more.

Junction of the fine head 20 and the ultra-fine pipe 30 is performed by, for example, adhering the pair of the connecting portions 220 to a tip end of the ultra-fine pipe 30 by using an adhesive.

Considering cell size (1 ∼ 100µm), a maximum width of the fine head 20 is preferably no greater than 100µm, more preferably no greater than 5µm so that cells are not destroyed upon insertion of the fine head 20 on a body as much as possible. In this embodiment, a maximum width of the fine head 20 is 1µm. Moreover, the fine head 20 is formed from a material having high permeability such as a magnetic material, for example, permalloy, silicon steel or the like.

The first head portion 200, for example, is shaped to taper toward a tip end thereof such as a substantially triangular pyramid, and provided with sides 200a, 200b, and 200c, a bottom surface 200d. The first head portion 200 is formed as it can make incisions in living tissue, that is, for example, a tip is formed at an acute angle. Incidentally, an overall shape of the fine head 20 may be such as prismatic or cylindrical if it is fine-sharp. A part of the first head portion 200 may be formed from a different material to material of other parts. For example, a tip portion may be formed from a material having low magnetic permeability, and other parts may be formed from a material having high magnetic permeability.

The first head portion 200 may have a protrusion such as a wire-shaped or blade-shaped as part.

An outer diameter of the fine head 20 is larger than an outer diameter of the ultra-fine pipe 30. Incidentally, an outer diameter of the fine head 20 may also be smaller than an outer diameter of the ultra-fine pipe 30.

Features of portions other than above portions are similar to the injection-suction apparatus according to the EXAMPLE 1.

The fine head 20 according to the EXAMPLE 2 can be used as a fine head 20 also in EXAMPLE 4 ∼ 10.

By means of the fine head 20 according to the EXAMPLE 2, it is possible to reduce a number of parts.

### EXAMPLE 3

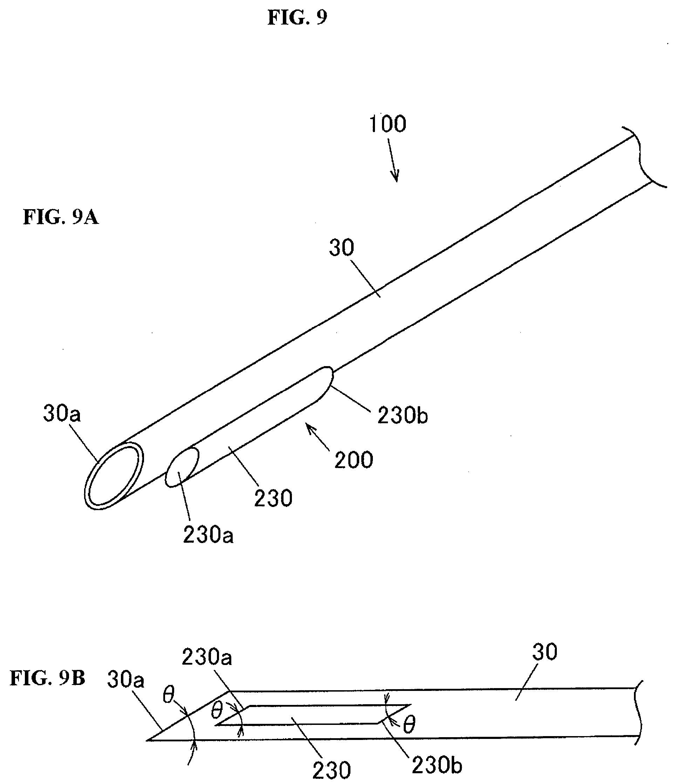

Figure 9 shows the injection-suction apparatus according to the EXAMPLE 3 of the embodiment of the present invention,

Figure 9A is a partial perspective view, Fig. 9B is a front view. The tip end of the fine head 20 of the injection-suction apparatus 100 is formed as it can make incisions in living tissue in the EXAMPLE 1, but the tip end of the ultra-fine pipe 2 is formed as it can make incisions in living tissue in this embodiment.

A cylindrical head portion 230 which is formed from a material having high permeability by using, for example, focused ion beam (FIB) constitutes the fine head 20 according to the EXAMPLE 3. A tip end and a rear end of the head portion 230 may be formed as it can make incisions in living tissue, that is, for example, may be provided with inclined surfaces 230a and 230b having an inclined angle θ of about 30 degrees. Incidentally, an overall shape of the fine head 20 may be such as prismatic if it is fine-sharp.

The tip end of the ultra-fine pipe 30 according to the EXAMPLE 3 may be formed as it can make incisions in living tissue, that is, for example, may be provided with an inclined surface 30a having an inclined angle θ of about 30 degrees. Junction of the fine head 20 and the ultra-fine pipe 30 is performed by, for example, adhering a peripheral surface of the head portion 230 to a peripheral surface of the ultra-fine pipe 30 by using an adhesive. A tip end of the head portion 230 is adhered being displaced to a rear end side from a tip end of the ultra-fine pipe 30 in this embodiment, but a tip end of the ultra-fine pipe 30 is adhered being displaced to a rear end side from a tip end of the head portion 230.

Considering cell size (1 ∼ 100µm), a maximum width of the fine head 20 is preferably no greater than 100µm, more preferably no greater than 5µm so that cells are not destroyed upon insertion of the fine head 20 on a body as much as possible. In this embodiment, a maximum width of the fine head 20 is 1µm. Moreover, the fine head 20 is formed from a material having high permeability such as a magnetic material, for example, permalloy, silicon steel or the like.

A part of the head portion 230 may be formed from a different material to material of other parts. For example, a tip portion and a rear portion may be formed from a material having low magnetic permeability, and other parts may be formed from a material having high magnetic permeability. The head portion 230 may have a protrusion such as a wire-shaped or blade-shaped as part.

For example, the ultra-fine pipe 30 is produced by forming from a material having low magnetic permeability using such as photolithography similar to such as a fabrication process of an inkjet printer nozzle, and cutting a tip of the ultra-fine pipe 30 obliquely using a micro-processing machine such as a focused ion beam processing machine.

The ultra-fine pipe 30 is attached to the fine head 20 in a state in which a tip end is opened, liquid such as an anti-cancer agent is injected or liquid such as cytosol is sucked through an opening portion in the tip end. The ultra-fine pipe 30 has, for example, an outer diameter of 100nm ∼ 1µm and a length of 5cm ∼ 10m. The ultra-fine pipe 30 is formed from a material having low permeability such as non-magnetic material, for example, aluminum, silver, gold, quartz glass or the like. However, gold is usually not suitable for a material of a film 2000, which will be described later. In this embodiment, the ultra-fine pipe 30 has an inner diameter of 150nm, an outer diameter of 350nm, a length of a size of more than half of the diameter of much human body such as 50cm.

A pump which is formed by using, for example, an electric motor or a piezoelectric element is connected to the ultra-fine pipe 30. In addition, an injection-suction apparatus 100 may has just the fine head 20 and the ultra-fine pipe 30 without connecting the pump. In this case, that may be an injection-suction apparatus which can inject a drug by a weight of the drug in a manner of infusion, suck urine by inserting into a bladder of which a pressure is high, or the like.

Features of portions other than above portions are similar to the injection-suction apparatus according to the EXAMPLE 1.

The fine head 20 and the ultra-fine pipe 30 according to the EXAMPLE 3 can be used as a fine head 20 and an ultra-fine pipe 30 also in EXAMPLE 4 ∼ 10.

By means of the fine head 20 and ultra-fine pipe 30 the according to the EXAMPLE 3, it is possible to simplify a configuration.

### EXAMPLE 4

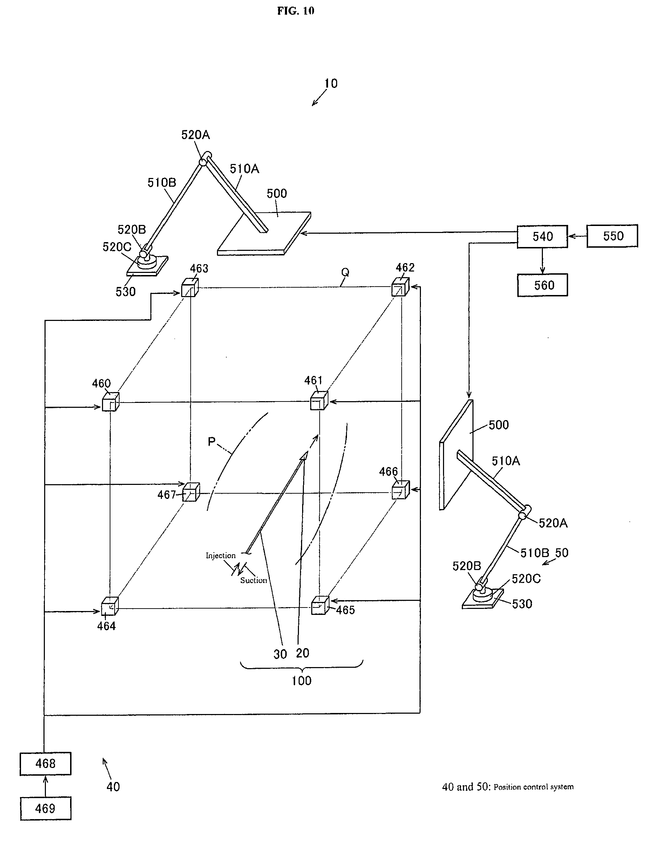

Figure 10 is a perspective view showing a configuration example of a schematic of an injection-suction system according to the EXAMPLE 4 of the embodiment of the present invention. The electromagnet 400; and the first arm 410A and the second arm 410B; and the first and second rotary drive units 420A and 420B; the third rotary drive unit 420C; and the base 430; and the control unit 440; and the operation unit 450 constitute the movement apparatus 40 in the EXAMPLE 1, but a plurality of electromagnets 460 to 467 each of which is provided in a predetermined position; and a control unit 468; and an operation unit 469 constitute a movement apparatus in this embodiment.

A movement apparatus 40 includes the electromagnets 460 to 467, the control unit 468 capable of controlling each part of the movement apparatus 40, the operation unit 469 that can indicate a three-dimensional position to which the fine head 20 moves to the control unit 468. The control unit 468 and the operation unit 469 are an example of a movement control unit of the movement apparatus being capable of controlling a magnitude and an orientation of a magnetic force that acts on the fine head in response to a magnetic field applied by the electromagnet.

Each of the electromagnets 460-467 generates a magnetic field of which magnitude is in accordance with a magnitude of a current conducted. The electromagnet 400 may be a superconducting magnet.

For example, the electromagnets 460-467 are provided so as to be positioned in each of vertices of a virtual cube Q surrounding a human body P. For example, each of the electromagnets 460-467 is provided in a direction such as a magnetic field which the electromagnet itself generates faces a position of a center of gravity of the virtual cube Q. In this case, the fine head 20 can move almost any position within the virtual cube Q.

Generally, a magnitude and an orientation of a resultant force of a magnetic force that acts on the fine head 20 is a magnitude and an orientation of a resultant force of a magnetic force that acts on the fine head 20 in response to a magnetic field applied by each of the electromagnets 460 to 467. For example, the fine head 20 is moved to a direction through a midpoint of a line connecting the electromagnet 460 and the electromagnet 461 when viewed from a current position of the fine head 20 if each of the electromagnet 460 and the electromagnet 461 out of eight electromagnets generate a magnetic field of which strength is same on condition that a distance between current positions of the fine head 20 and the electromagnet 460 equals to a distance between current positions of fine head 20 and the electromagnet 461; and directions of the electromagnet 460 and the electromagnet 461 are directions such as a magnetic field which each of the electromagnet 460 and the electromagnet 461 generates faces a current position of the fine head 20; and an angle defined by an orientation of a magnetic field which the electromagnet 460 generates and an orientation of a magnetic field which the electromagnet 461 generates is 120 degrees. The magnitude of the resultant force of the magnetic force that acts on the fine head 20 then is same as a magnitude of a magnetic force that acts on the fine head 20 in response to a magnetic field which the electromagnet 460 or the electromagnet 461 generates.

For example, the electromagnets 460-467 are provided so as to be positioned in each of vertices of such as tetrahedron virtual surrounding a human body P. For example, each of the electromagnets 460-467 is provided in a direction such as a magnetic field which the electromagnet itself generates faces a position of a center of gravity of such as the tetrahedron virtual. In this case, the fine head 20 can move almost any position within such as the tetrahedron virtual.

The operation unit 450 is configured to indicate a three-dimensional position to which the fine head 20 moves by, for example, lever operation or computer.

The control unit 440 moves the fine head 20 in a direction of indicated position i.e. target position based on a three-dimensional position indicated by the operation unit 450, by controlling a magnitude and an orientation of a resultant force of a magnetic force that acts on the fine head 20 by finely adjusting a strength of a magnetic field generated by each of the electromagnets 460-467 by controlling a magnitude of a current conducted to each of the electromagnets 460-467.

For example, by performing control of an advancement direction of the fine head 20 to adjust a magnitude and a position and an orientation of the magnetic field applied to the fine head 20 by each of the electromagnets 460-467, that is, to adjust a magnitude and an orientation of the resultant force of the magnetic force that acts on the fine head 20 so as to suppress a bending of a movement path of the ultra-fine pipe 30, that is, as the ultra-fine pipe 30 is inserted substantially linearly into a target position, the ultra-fine pipe 30 does not get tangled, and a body tissue is not damaged so much when the fine head 20 and the ultra-fine pipe 30 are inserted into a body or are pulled out from a body. That is, problems such as the ultra-fine pipe 30 knots and the knot damages a body tissue when to move, and an internal circumference side of a curve increases power and a body tissue is cut when it is pulled to one direction in spite of orbit bending if the ultra-fine pipe 30 is very fine and the pipe in itself is sharp like a thread-like knife are hard to occur. In addition, in this case, advantage that it becomes easier to let the fine head 20 reach to a target position quickly because a movement distance up to the target position of the fine head 20 is shortened is obtained. Incidentally, the state is also included in a state that the ultra-fine pipe 30 has been inserted substantially linearly into a target position, to the extent that ultra-fine pipe 30 can not damage a body tissue so much for the reasons mentioned above, even if there is fold or bent or the like on a movement path of the ultra-fine pipe 30.

Position control of the fine head 20 by the control unit 440, for example, may be a simply control like that the fine head 20 is pulled in a direction of a target position, or a control like that it goes back to left if it goes to right too much, it goes back to right if it goes to left too much, it goes back to below if it goes to above too much, it goes back to above if it goes to below too much (see Figure 17). In particular, in a case of a control such as 'it goes back to left if it goes to right too much, it goes back to right if it goes to left too much, it goes back to below if it goes to above too much, it goes back to above if it goes to below too much', the fine head 20 becomes easier to reach a target position accurately even in a body of a human or animal having a complicated structure because when there is a deviation, the deviation can be corrected naturally.

Moreover, the fine head 20 and the ultra-fine pipe 30 may be put in a container filled with water prior to use and the container with them may be adhered to a patient's body in use, a tip end of the ultra-fine pipe 30 may be moved by the movement apparatus 40 from there.

Features of portions other than above portions are similar to the injection-suction system according to the EXAMPLE 1.

The movement apparatus 40 and the position detection apparatus 50 are an example of the position control system of the embodiment of the present invention.

The movement apparatus 40 according to the EXAMPLE 4 can be used as a movement apparatus 40 also in EXAMPLE 2,3,5 ∼ 10.

By means of the movement apparatus 40 according to the EXAMPLE 4, a maintenance is facilitated in many cases because moving parts are reduced by eliminating the arms.

### EXAMPLE 5

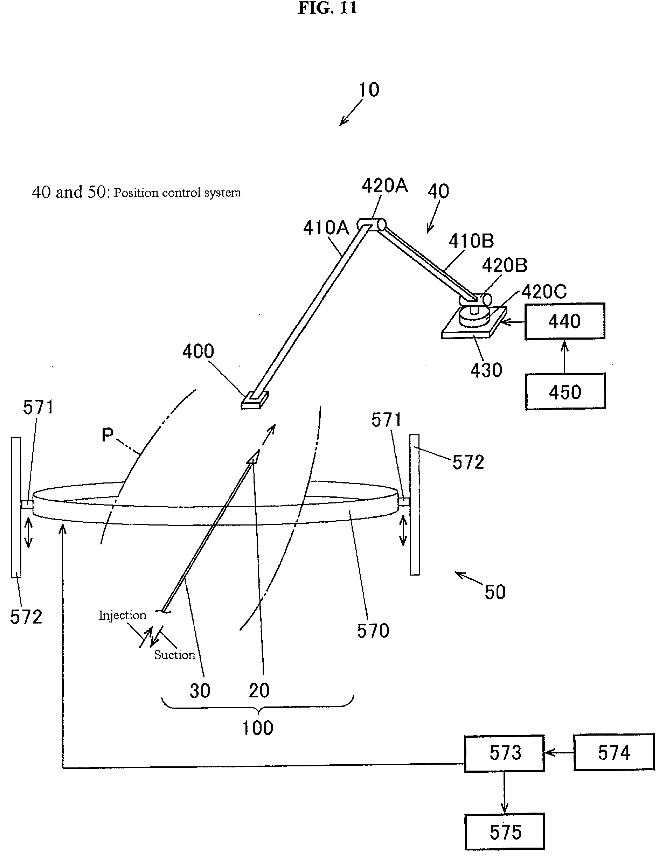

Figure 11 is a perspective view showing a configuration example of a schematic of an injection-suction system according to the EXAMPLE 5 of the embodiment of the present invention. The X-ray CCD sensors 500 constitute the radiographic imaging sensor which a imaging unit of the position detection apparatus 50 has in the EXAMPLE 1, but a MRI sensor 570 constitute a radiographic imaging sensor which a imaging unit of a position detection apparatus 50 has in this embodiment.

The position detection apparatus 50 includes an MRI sensor 570 capable of photographing a mark made of a MRI contrast agent applied to the fine head 20, a slide drive unit 571 which can linearly move the MRI sensor 570 in a direction perpendicular to an imaging surface, a base 572 which supports the slide drive unit 571, a control unit 573 capable of driving and controlling the slide drive unit 571 and detecting a position of the fine head 20, an operation unit 574 that can indicate a three-dimensional position of the MRI sensor 570, a display unit 575 that can display detected position of the fine head 20. A position of forming the mark of a MRI contrast agent may be also in a tip end side position of the ultra-fine pipe 30. The display unit 575 may be omitted if a computer automatically peforms confirming that the fine head 20 has reached a target position. The MRI sensor 570 is an example of a radiographic imaging sensor which an imaging unit of the position detection apparatus has.

A three-dimensional position of the fine head 20 can be known by repeating photographing by nuclear magnetic resonance imaging while the MRI sensor 570 is moved little by little to a direction perpendicular to the imaging surface, that is, photographing a plurality of image data of which imaging surfaces are parallel with each other by the MRI sensor 570; and searching the image data for a portion corresponding to the mark by an image recognition processing.

Just the mark is projected in the MRI sensor 570 and a position detection of the mark becomes easy when a material of the mark has an atom which is not present in a body.

It is preferable that a magnitude of a static magnetic field and a gradient magnetic field to be applied to a body is to the extent that the fine head 20 hardly moves. In general, a resolution of the MRI sensor decreases as the lower the magnitude of a static magnetic field and a gradient magnetic field to be applied to a body, but a position detection of the mark becomes easy even in a weak magnetic field since, for example, just the mark is projected in the MRI sensor when a material of the mark has an atom which is not present in a body.

It is preferred that the electromagnet 400 which the movement apparatus 40 has does not produce a magnetic field in photographing by the MRI sensor 570 because a position detection by the MRI sensor 570 is hindered.

The slide drive unit 571 is connected to the MRI sensor 570, and move the MRI sensor 570 to a direction perpendicular to an imaging surface.

The operation unit 574 is configured to indicate a position to which the MRI sensor 570 moves by, for example, lever operation or computer.

The position detection apparatus 50 is configured so as to measure a three-dimensional position which shows a reference position (eg, a predetermined position existing on a boundary between the MRI sensor 570 and an imaging surface) of the imaging surface of the MRI sensor 570 precisely (eg, in micrometer units) by providing a position sensor between the MRI sensor 570 and the slide drive unit 571. That position sensor is an example of a position detection unit for a radiographic imaging sensor capable of measuring a three-dimensional position of the radiographic imaging sensor with an imaging unit of the position detection apparatus.

The control unit 573 is an example of a position detection unit. The control unit 573 includes an image recognition processing unit capable of performing image recognition processing that can recognize images by a pattern recognition.

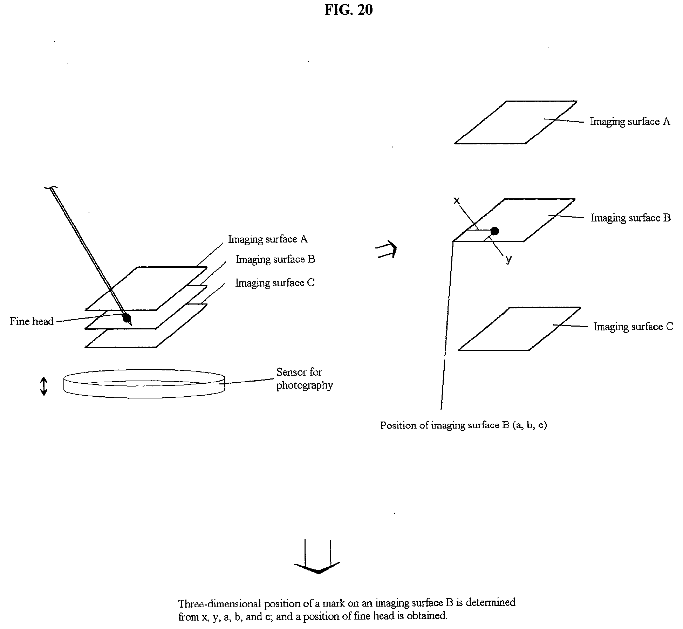

The control unit 573, for example, detects a position of the fine head 20 in the following procedure (see Figure 20):
1. Preparing a plurality of image data of which imaging surfaces are parallel with each other using the MRI sensor 570;
2. Determining a two-dimensional position of a mark within an imaging range of the image data by searching the image data for a portion corresponding to the mark using the image recognition processing unit; and
3. Obtaining a position of the fine head 20 by adding a three-dimensional position which shows a reference position of the imaging surface of the MRI sensor 570 to the two-dimensional position of the mark in the imaging range of image data. Incidentally, in 2, if the mark is found in a plurality of image data of which imaging surfaces are adjacent to each other, it may be treated as the mark is found in image data of which imaging surface is center among those of the plurality of image data.

The control unit 573 moves the MRI sensor 570 in a direction of indicated position based on a three-dimensional position indicated by the operation unit 574 by controlling the slide drive unit 571.

A material of the mark may be a material of which nuclear spin is except for 0, other than a MRI contrast agent.

Incidentally, the mark is better to be in a tip end side position of the fine head 20 or the ultra-fine pipe 30, and the mark need not be formed separately from the fine head 20 and the ultra-fine pipe 30. In other words, it may be possible that a material of the fine head 20 is a substance capable of transmitting photographed by the MRI sensor 570, and the fine head 20 itself is treated as the mark. For example, a MRI contrast agent may be kneaded to the material of the fine head 20. In addition, it may be possible that a material of the ultra-fine pipe 30 is a substance capable of transmitting photographed by the MRI sensor 570, and a total image or a wide range of image including a tip end side of the ultra-fine pipe 30 is projected in a photographed image data in the MRI sensor 570, and a portion corresponding to the tip end side in a image of the ultra-fine pipe 30 is treated as an image of the mark in the image recognition processing. For example, it may be possible that the ultra-fine pipe 30 is made kneading a MRI contrast agent to a material of the ultra-fine pipe 30.

In particular when a material of the ultra-fine pipe 30 is a substance capable of transmitting photographed by the MRI sensor 570, and a total image or a wide range of image including a tip end side of the ultra-fine pipe 30 is projected in a photographed image data in the MRI sensor 570, and a portion corresponding to the tip end side in a image of the ultra-fine pipe 30 is treated as an image of the mark in the image recognition processing, the control unit 573 may detect a position of the fine head 20 in the following procedure:
1. Preparing a plurality of image data of which imaging surfaces are parallel with each other using the MRI sensor 570;
2. Determining a two-dimensional position of a mark within an imaging range of the image data by searching the image data for a portion corresponding to the ultra-fine pipe 30 using the image recognition processing unit, and a portion which is found in image data of an outermost side of a body that is not at a root side of the ultra-fine pipe 30 is treated as a portion corresponding to the mark among found portions corresponding to the ultra-fine pipe 30; and
3. Obtaining a position of the fine head 20 by adding a three-dimensional position which shows a reference position of the imaging surface of the MRI sensor 570 to the two-dimensional position of the mark in the imaging range of image data.

For example, if each of photographing positions is numbered in ascending order from a root side or the reverse side of the ultra-fine pipe 30, it can be performed easily by simply comparing the numbers that which of the image data is the one of an outermost side of a body that is not at a root side of the ultra-fine pipe 30. In general, this procedure is used when the fine head 20 is moved by the movement apparatus 40 so that the ultra-fine pipe 30 is inserted substantially linearly into a target position. Incidentally, this procedure can not be used as is usually when a direction of the ultra-fine pipe 30 is parallel to the imaging surface of the MRI sensor 570, but a two-dimensional position of a mark within an imaging range of the image data can be determined by searching the image data in which the ultra-fine tube 30 is projected for a portion corresponding to a tip end side in a image of the ultra-fine pipe 30 by the image recognition processing unit in 2, that is, searching the image data prepared in 1 for a portion corresponding to the mark by the image recognition processing unit.

In particular when a material of the ultra-fine pipe 30 is a substance capable of transmitting photographed by the MRI sensor 570; and a total image or a wide range of image including a tip end side of the ultra-fine pipe 30 is projected in a photographed image data in the MRI sensor 570; and a portion corresponding to the tip end side in a image of the ultra-fine pipe 30 is treated as an image of the mark in the image recognition processing, it may be possible that the position detection apparatus 50 is provided with a cross-sectional images three-dimensional imaging processing unit for producing three-dimensional image data by interpolating between a plurality of cross-sectional image data of which imaging surfaces are parallel with each other, and a three-dimensionally image recognition processing is performed by the image recognition processing unit, and the control unit 573 detects a position of the fine head 20 in the following procedure:
1. Preparing three-dimensional image data, produced from a plurality of image data of which imaging surfaces are parallel with each other which are photographed by the MRI sensor 570, using the cross-sectional images three-dimensional imaging processing unit;
2. Determining a three-dimensional position of a mark within an imaging range of the three-dimensional image data by treating a portion corresponding to a tip end side in a image of the ultra-fine pipe 30 which is projected in the three-dimensional image data as an image of a mark, and searching the three-dimensional image data for a portion corresponding to a mark using the imaging processing unit; and
3. Obtaining a position of the fine head 20 by adding a three-dimensional position which shows a reference position (such as a reference position of an imaging range of the MRI sensor 570 at the time of locating at an end of a movement range) of an imaging range of the MRI sensor 570 to a three-dimensional position of a mark in an imaging range of the three-dimensional image data.

Incidentally, a three-dimensionally image recognition processing may be a process using an Interactive Closed Point algorithm or
a process in which mappings from an infinite distance point of view in two orthogonal directions are provided, and a two-dimensionally image recognition processing is performed in each of the mappings, and the results are synthesized in a orthogonal direction.

Features of portions other than above portions are similar to the injection-suction apparatus according to the EXAMPLE 1.

The movement apparatus 40 and the position detection apparatus 50 are an example of the position control system of the embodiment of the present invention.

The position detection apparatus 50 according to the EXAMPLE 5 can be used as a position detection apparatus 50 also in EXAMPLE 2 ∼ 4.

By means of the position detection apparatus 50 according to the EXAMPLE 5, radiation is not required and a safety is improved, and a maintenance is facilitated in many cases because moving parts are reduced by eliminating the arms.

### EXAMPLE 6

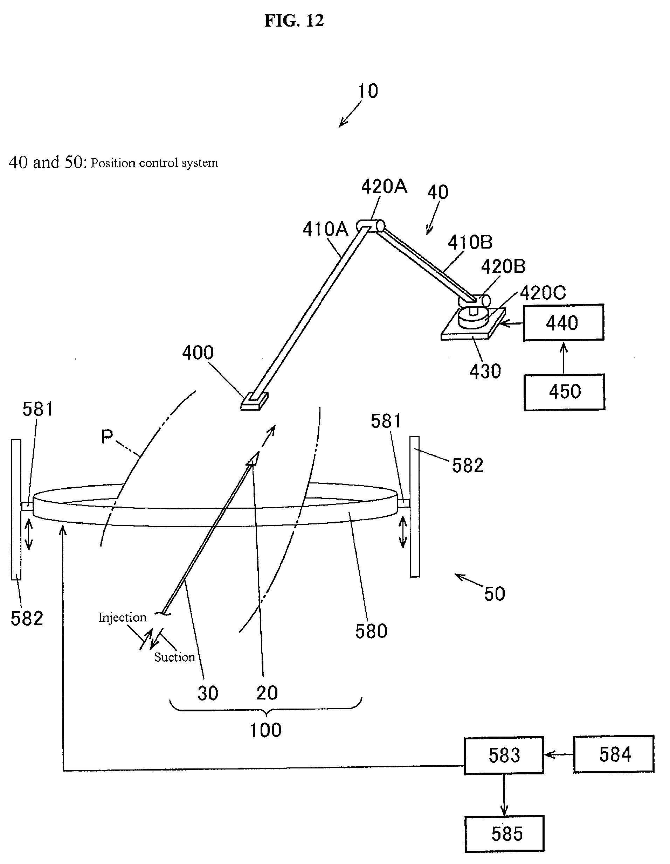

Figure 12 is a perspective view showing a configuration example of a schematic of an injection-suction system according to the EXAMPLE 5 of the embodiment of the present invention. The X-ray CCD sensors 500 constitute the radiographic imaging sensor which a imaging unit of the position detection apparatus 50 has in the EXAMPLE 1, but a CT sensor 580 constitute a radiographic imaging sensor which a imaging unit of a position detection apparatus 50 has in this embodiment.

The position detection apparatus 50 includes an CT sensor 580 capable of photographing a mark made of a CT contrast agent applied to the fine head 20, a slide drive unit 581 which can linearly move the CT sensor 580 in a direction perpendicular to an imaging surface, a base 582 which supports the slide drive unit 581, a control unit 583 capable of driving and controlling the slide drive unit 581 and detecting a position of the fine head 20, an operation unit 584 that can indicate a three-dimensional position of the CT sensor 580, a display unit 585 that can display detected position of the fine head 20. A position of forming the mark of a CT contrast agent may be also in a tip end side position of the ultra-fine pipe 30. The display unit 585 may be omitted if a computer automatically peforms confirming that the fine head 20 has reached a target position. The CT sensor 580 is an example of a radiographic imaging sensor which an imaging unit of the position detection apparatus has.

A three-dimensional position of the fine head 20 can be known by repeating photographing by computed tomography imaging while the CT sensor 580 is moved little by little to a direction perpendicular to the imaging surface, that is, photographing a plurality of image data of which imaging surfaces are parallel with each other by the CT sensor 580; and searching the image data for a portion corresponding to the mark by an image recognition processing.

CT which is used in this embodiment may be X-ray CT, positron emission tomography (PET), single photon emission tomography (SPECT), and an ultrasonic CT.

The slide drive unit 581 is connected to the CT sensor 580, and move the CT sensor 580 to a direction perpendicular to an imaging surface.

The operation unit 584 is configured to indicate a position to which the CT sensor 580 moves by, for example, lever operation or computer.

The position detection apparatus 50 is configured so as to measure a three-dimensional position which shows a reference position (eg, a predetermined position existing on a boundary between the CT sensor 580 and an imaging surface) of the imaging surface of the CT sensor 580 precisely (eg, in micrometer units) by providing a position sensor between the CT sensor 580 and the slide drive unit 581. That position sensor is an example of a position detection unit for a radiographic imaging sensor capable of measuring a three-dimensional position of the radiographic imaging sensor with an imaging unit of the position detection apparatus.

The control unit 583 is an example of a position detection unit. The control unit 583 includes an image recognition processing unit capable of performing image recognition processing that can recognize images by a pattern recognition.

The control unit 583, for example, detects a position of the fine head 20 in the following procedure (see Figure 20):
1. Preparing a plurality of image data of which imaging surfaces are parallel with each other using the CT sensor 580;
2. Determining a two-dimensional position of a mark within an imaging range of the image data by searching the image data for a portion corresponding to the mark using the image recognition processing unit; and
3. Obtaining a position of the fine head 20 by adding a three-dimensional position which shows a reference position of the imaging surface of the CT sensor 580 to the two-dimensional position of the mark in the imaging range of image data. Incidentally, in 2, if the mark is found in a plurality of image data of which imaging surfaces are adjacent to each other, it may be treated as the mark is found in image data of which imaging surface is center among those of the plurality of image data.

The control unit 583 moves the CT sensor 580 in a direction of indicated position based on a three-dimensional position indicated by the operation unit 584 by controlling the slide drive unit 581.

A material of the mark may be a substance capable of transmitting photographed by the CT sensor 580, other than a CT contrast agent.

Incidentally, the mark is better to be in a tip end side position of the fine head 20 or the ultra-fine pipe 30, and the mark need not be formed separately from the fine head 20 and the ultra-fine pipe 30. In other words, it may be possible that a material of the fine head 20 is a substance capable of transmitting photographed by the CT sensor 580, and the fine head 20 itself is treated as the mark. For example, a CT contrast agent may be kneaded to the material of the fine head 20. In addition, it may be possible that a material of the ultra-fine pipe 30 is a substance capable of transmitting photographed by the CT sensor 580, and a total image or a wide range of image including a tip end side of the ultra-fine pipe 30 is projected in a photographed image data in the CT sensor 580, and a portion corresponding to the tip end side in a image of the ultra-fine pipe 30 is treated as an image of the mark in the image recognition processing. For example, it may be possible that the ultra-fine pipe 30 is made kneading a CT contrast agent to a material of the ultra-fine pipe 30.

In particular when a material of the ultra-fine pipe 30 is a substance capable of transmitting photographed by the CT sensor 580, and a total image or a wide range of image including a tip end side of the ultra-fine pipe 30 is projected in a photographed image data in the CT sensor 580, and a portion corresponding to the tip end side in a image of the ultra-fine pipe 30 is treated as an image of the mark in the image recognition processing, the control unit 583 may detect a position of the fine head 20 in the following procedure:
1. Preparing a plurality of image data of which imaging surfaces are parallel with each other using the CT sensor 580;
2. Determining a two-dimensional position of a mark within an imaging range of the image data by searching the image data for a portion corresponding to the ultra-fine pipe 30 using the image recognition processing unit, and a portion which is found in image data of an outermost side of a body that is not at a root side of the ultra-fine pipe 30 is treated as a portion corresponding to the mark among found portions corresponding to the ultra-fine pipe 30; and
3. Obtaining a position of the fine head 20 by adding a three-dimensional position which shows a reference position of the imaging surface of the CT sensor 580 to the two-dimensional position of the mark in the imaging range of image data.

For example, if each of photographing positions is numbered in ascending order from a root side or the reverse side of the ultra-fine pipe 30, it can be performed easily by simply comparing the numbers that which of the image data is the one of an outermost side of a body that is not at a root side of the ultra-fine pipe 30. In general, this procedure is used when the fine head 20 is moved by the movement apparatus 40 so that the ultra-fine pipe 30 is inserted substantially linearly into a target position. Incidentally, this procedure can not be used as is usually when a direction of the ultra-fine pipe 30 is parallel to the imaging surface of the CT sensor 580, but a two-dimensional position of a mark within an imaging range of the image data can be determined by searching the image data in which the ultra-fine tube 30 is projected for a portion corresponding to a tip end side in a image of the ultra-fine pipe 30 by the image recognition processing unit in 2, that is, searching the image data prepared in 1 for a portion corresponding to the mark by the image recognition processing unit.

In particular when a material of the ultra-fine pipe 30 is a substance capable of transmitting photographed by the CT sensor 580; and a total image or a wide range of image including a tip end side of the ultra-fine pipe 30 is projected in a photographed image data in the CT sensor 580; and a portion corresponding to the tip end side in a image of the ultra-fine pipe 30 is treated as an image of the mark in the image recognition processing, it may be possible that the position detection apparatus 50 is provided with a cross-sectional images three-dimensional imaging processing unit for producing three-dimensional image data by interpolating between a plurality of cross-sectional image data of which imaging surfaces are parallel with each other, and a three-dimensionally image recognition processing is performed by the image recognition processing unit, and the control unit 583 detects a position of the fine head 20 in the following procedure:
1. Preparing three-dimensional image data, produced from a plurality of image data of which imaging surfaces are parallel with each other which are photographed by the CT sensor 580, using the cross-sectional images three-dimensional imaging processing unit;
2. Determining a three-dimensional position of a mark within an imaging range of the three-dimensional image data by treating a portion corresponding to a tip end side in a image of the ultra-fine pipe 30 which is projected in the three-dimensional image data as an image of a mark, and searching the three-dimensional image data for a portion corresponding to a mark using the imaging processing unit; and
3. Obtaining a position of the fine head 20 by adding a three-dimensional position which shows a reference position (such as a reference position of an imaging range of the CT sensor 580 at the time of locating at an end of a movement range) of an imaging range of the CT sensor 580 to a three-dimensional position of a mark in an imaging range of the three-dimensional image data.

Incidentally, a three-dimensionally image recognition processing may be a process using an Interactive Closed Point algorithm or
a process in which mappings from an infinite distance point of view in two orthogonal directions are provided, and a two-dimensionally image recognition processing is performed in each of the mappings, and the results are synthesized in a orthogonal direction.

Features of portions other than above portions are similar to the injection-suction apparatus according to the EXAMPLE 1.

The movement apparatus 40 and the position detection apparatus 50 are an example of the position control system of the embodiment of the present invention.

The position detection apparatus 50 according to the EXAMPLE 6 can be used as a position detection apparatus 50 also in EXAMPLE 2 ∼ 4.

By means of the position detection apparatus 50 according to the EXAMPLE 6, it becomes generally easier to obtain a higher resolution than the EXAMPLE 5 in which a magnetic field to be used is limited to very weak one, and a maintenance is facilitated in many cases because moving parts are reduced by eliminating the arms.

### EXAMPLE 7

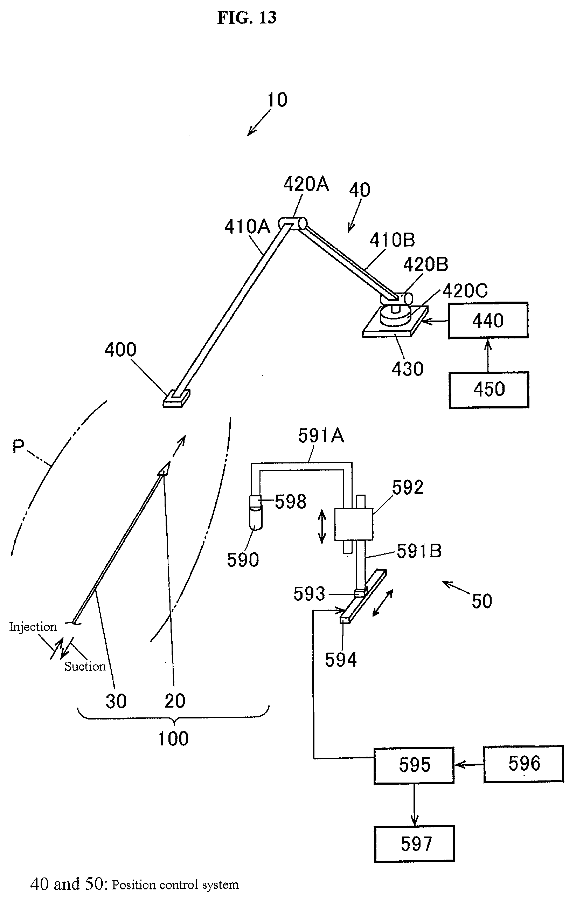

Figure 13 is a perspective view showing a configuration example of a schematic of an injection-suction system according to the EXAMPLE 5 of the embodiment of the present invention. The X-ray CCD sensors 500 constitute the radiographic imaging sensor which a imaging unit of the position detection apparatus 50 has in the EXAMPLE 1, but an ultrasonic inspection probe 590 constitute a radiographic imaging sensor which a imaging unit of a position detection apparatus 50 has in this embodiment.

The position detection apparatus 50 includes an ultrasonic inspection probe 590 capable of generating an ultrasonic wave in fan shape with respect to the fine head 20 serving also as a mark and receiving a reflected ultrasonic wave, first arm 591 A for supporting the ultrasonic inspection probe 590, extension/contraction drive unit 592 capable of moving the first arm 591 A in a vertical direction, second arm 591B for supporting the extension/contraction drive unit 592 and a slide drive unit 593, a slide drive unit 593 which can move the second arm 591 B in a horizontal direction, a base 594 which supports the slide drive unit 593, a piezoelectric element 598 capable of measuring a pressure between the ultrasonic inspection probe 590 and the first arm 591A, a control unit 595 capable of driving and controlling the extension/contraction drive unit 592 and the slide drive unit 593; and detecting a position of the fine head 20, an operation unit 596 that can indicate a three-dimensional position of the ultrasonic inspection probe 590, a display unit 597 that can display detected position of the fine head 20. The display unit 597 may be omitted if a computer automatically peforms confirming that the fine head 20 has reached a target position. The ultrasonic inspection probe 590 is an example of a radiographic imaging sensor which an imaging unit of the position detection apparatus has.

The ultrasonic inspection probe 590 can generate an acoustic wave in a fan shape, and see a cross-sectional image of a body.

A three-dimensional position of the fine head 20 can be known by repeating photographing by ultrasonic inspection while the ultrasonic inspection probe 590 is moved little by little to a direction perpendicular to the imaging surface, that is, photographing a plurality of image data of which imaging surfaces are parallel with each other by the ultrasonic inspection probe 590; and searching the image data for a portion corresponding to the mark by an image recognition processing.

The mark is preferably formed from a material having features such as the mark is projected in the photographed image data so that it is easy to distinguish the mark from body tissues and the ultra-fine pipe 30, that is, an acoustic impedance is sufficiently separated.

The slide drive unit 593 is connected to the ultrasonic inspection probe 590 via an arm, and move the ultrasonic inspection probe 590 to a direction perpendicular to an imaging surface.

The piezoelectric element 598 is attached between the ultrasonic inspection probe 590 and the first arm 591 A.

The operation unit 596 is configured to indicate a position to which the ultrasonic inspection probe 590 moves by, for example, lever operation or computer.

The position detection apparatus 50 is configured so as to measure a three-dimensional position which shows a reference position (eg, a predetermined position existing on a boundary between the ultrasonic inspection probe 590 and an imaging surface) of the imaging surface of the ultrasonic inspection probe 590 precisely (eg, in micrometer units) by providing a position sensor between the first arm 591 A and the extension/contraction drive unit 592, between the second arm 591 B and the slide drive unit 593 respectively. That position sensor is an example of a position detection unit for a radiographic imaging sensor capable of measuring a three-dimensional position of the radiographic imaging sensor with an imaging unit of the position detection apparatus.

The control unit 595 is an example of a position detection unit. The control unit 595 includes an image recognition processing unit capable of performing image recognition processing that can recognize images by a pattern recognition.

The control unit 595, for example, detects a position of the fine head 20 in the following procedure (see Figure 20):
1. Preparing a plurality of image data of which imaging surfaces are parallel with each other using the ultrasonic inspection probe 590;
2. Determining a two-dimensional position of a mark within an imaging range of the image data by searching the image data for a portion corresponding to the mark using the image recognition processing unit; and
3. Obtaining a position of the fine head 20 by adding a three-dimensional position which shows a reference position of the imaging surface of the ultrasonic inspection probe 590 to the two-dimensional position of the mark in the imaging range of image data.

Incidentally, in 2, if the mark is found in a plurality of image data of which imaging surfaces are adjacent to each other, it may be treated as the mark is found in image data of which imaging surface is center among those of the plurality of image data.

The control unit 595 moves the ultrasonic inspection probe 590 to an indicated position based on a three-dimensional position indicated by the operation unit 596, by controlling the extension/contraction drive unit 592 and the slide drive unit 593. A movement of the ultrasonic inspection probe 590 is preferably performed while checking whether a magnitude of a force for pressing the ultrasonic inspection probe 590 to a body is an appropriate size (not too high or not too low). For example, a movement of the ultrasonic inspection probe 590 is performed in the following procedure:
1. The second arm 591 B is Slightly moved in a direction of a position indicated by the operation unit 596 by adjusting the slide drive unit 593.
2. A pressure between the ultrasonic inspection probe 590 and the first arm 591A is measured by the piezoelectric element 598. The ultrasonic inspection probe 590 is slightly moved in a direction of a body by adjusting the extension/contraction drive unit 592 if the pressure is too low, next the instruction of 2 is repeated from first. The ultrasonic inspection probe 590 is slightly moved in a direction opposite to a direction of a body by adjusting the extension/contraction drive unit 592 if the pressure is too high, next the instruction of 2 is repeated from first. If the pressure is appropriately sized, it is confirmed whether a position of the ultrasonic inspection probe 590 has reached the position indicated by the operation unit 596. If reached, assuming that the movement has completed. If not reached, the instruction of 1 is repeated.

Incidentally, the ultrasonic inspection probe 590 may be manually moved to a position in which the ultrasonic inspection probe 590 is in contact with a body with pressure of which size is near proper by doctor in advance.

Incidentally, the mark is better to be in a tip end side position of the fine head 20 or the ultra-fine pipe 30, and the mark may be formed separately from the fine head 20 and the ultra-fine pipe 30. For example, it may be possible that a material which can reflect an ultrasonic wave of used frequency easily is applied to the fine head 20, and that is treated as the mark. In addition, it may be possible that a material of the ultra-fine pipe 30 is a substance capable of transmitting photographed by the ultrasonic inspection probe 590, and a total image or a wide range of image including a tip end side of the ultra-fine pipe 30 is projected in a photographed image data in the ultrasonic inspection probe 590, and a portion corresponding to the tip end side in a image of the ultra-fine pipe 30 is treated as an image of the mark in the image recognition processing.

In particular when a material of the ultra-fine pipe 30 is a substance capable of transmitting photographed by the ultrasonic inspection probe 590, and a total image or a wide range of image including a tip end side of the ultra-fine pipe 30 is projected in a photographed image data in the ultrasonic inspection probe 590, and a portion corresponding to the tip end side in a image of the ultra-fine pipe 30 is treated as an image of the mark in the image recognition processing, the control unit 595 may detect a position of the fine head 20 in the following procedure:
1. Preparing a plurality of image data of which imaging surfaces are parallel with each other using the ultrasonic inspection probe 590;
2. Determining a two-dimensional position of a mark within an imaging range of the image data by searching the image data for a portion corresponding to the ultra-fine pipe 30 using the image recognition processing unit, and a portion which is found in image data of an outermost side of a body that is not at a root side of the ultra-fine pipe 30 is treated as a portion corresponding to the mark among found portions corresponding to the ultra-fine pipe 30; and
3. Obtaining a position of the fine head 20 by adding a three-dimensional position which shows a reference position of the imaging surface of the ultrasonic inspection probe 590 to the two-dimensional position of the mark in the imaging range of image data.

For example, if each of photographing positions is numbered in ascending order from a root side or the reverse side of the ultra-fine pipe 30, it can be performed easily by simply comparing the numbers that which of the image data is the one of an outermost side of a body that is not at a root side of the ultra-fine pipe 30. In general, this procedure is used when the fine head 20 is moved by the movement apparatus 40 so that the ultra-fine pipe 30 is inserted substantially linearly into a target position. Incidentally, this procedure can not be used as is usually when a direction of the ultra-fine pipe 30 is parallel to the imaging surface of the ultrasonic inspection probe 590, but a two-dimensional position of a mark within an imaging range of the image data can be determined by searching the image data in which the ultra-fine tube 30 is projected for a portion corresponding to a tip end side in a image of the ultra-fine pipe 30 by the image recognition processing unit in 2, that is, searching the image data prepared in 1 for a portion corresponding to the mark by the image recognition processing unit.

In particular when a material of the ultra-fine pipe 30 is a substance capable of transmitting photographed by the ultrasonic inspection probe 590; and a total image or a wide range of image including a tip end side of the ultra-fine pipe 30 is projected in a photographed image data in the ultrasonic inspection probe 590; and a portion corresponding to the tip end side in a image of the ultra-fine pipe 30 is treated as an image of the mark in the image recognition processing, it may be possible that the position detection apparatus 50 is provided with a cross-sectional images three-dimensional imaging processing unit for producing three-dimensional image data by interpolating between a plurality of cross-sectional image data of which imaging surfaces are parallel with each other, and a three-dimensionally image recognition processing is performed by the image recognition processing unit, and the control unit 595 detects a position of the fine head 20 in the following procedure:
1. Preparing three-dimensional image data, produced from a plurality of image data of which imaging surfaces are parallel with each other which are photographed by the ultrasonic inspection probe 590, using the cross-sectional images three-dimensional imaging processing unit;
2. Determining a three-dimensional position of a mark within an imaging range of the three-dimensional image data by treating a portion corresponding to a tip end side in a image of the ultra-fine pipe 30 which is projected in the three-dimensional image data as an image of a mark, and searching the three-dimensional image data for a portion corresponding to a mark using the imaging processing unit; and
3. Obtaining a position of the fine head 20 by adding a three-dimensional position which shows a reference position (such as a reference position of an imaging range of tthe ultrasonic inspection probe 590 at the time of locating at an end of a movement range) of an imaging range of the ultrasonic inspection probe 590 to a three-dimensional position of a mark in an imaging range of the three-dimensional image data.

Incidentally, a three-dimensionally image recognition processing may be a process using an Interactive Closed Point algorithm or
a process in which mappings from an infinite distance point of view in two orthogonal directions are provided, and a two-dimensionally image recognition processing is performed in each of the mappings, and the results are synthesized in a orthogonal direction.

A range which is marked by doctor hand or the like is in a range of accuracy of millimeters at most though it varies depending on a manual dexterity of the doctor corresponding to the work. In addition, the fine head 20 is just moved to 'generally' near center of the range in many cases. Therefore, it is possible to use large X-ray CCD sensors for digital X-ray imaging which are common that accuracy is low to reduce a cost because they are so huge as the position detection apparatus 50. It is also possible to fix the X-ray CCD sensors in a predetermined position without using the arms until a treatment is finished when using large X-ray CCD sensors for digital X-ray imaging as the position detection apparatus 50. A maintenance is facilitated in many cases because moving parts are reduced by eliminating the arms. In the case the fine head 20 is desired to move inside or outside of a cell membrane certainly, a movement of the fine head 20 with high accuracy is required, but an accuracy of a position detection may be also lower since it is possible to determine whether the tip end of the ultra-fine pipe 30 is inside or outside of a cell membrane by a method such as measuring a pressure by attaching a pressure gauge to the pump. Therefore, it is possible to use the large X-ray CCD sensors for digital X-ray imaging as the position detection apparatus 50 even in this case.

The position detection apparatus 50 may be provided with a rotary drive unit in between the piezoelectric element 598 and the first arm 591 A.

Moreover, it is also possible to use an X-ray CMOS sensors in place of the X-ray CCD sensors.

The position detection apparatus 50 may be provided with other slide drive unit which can move the base 594 in a direction perpendicular to a moving direction of the slide drive unit 593.

It may be possible that the ultrasonic inspection probe 590 having the three-dimensional position sensor (shch as sensor using a plurality of radio rangefinders or sensor photographing an infrared pattern with an infrared sensor) is moved by doctor hand instead of using the first arm 591 A, the extension/contraction drive unit 592, the second arm 591 B, the slide drive unit 593, the base 594, the piezoelectric element 598, and the operation unit 596. In that case, the control unit 595 may be capable of indicating a direction in which the ultrasonic inspection probe 590 moves by such as images or sounds. Features of portions other than above portions are similar to the injection-suction system according to the EXAMPLE 1.

The movement apparatus 40 and the position detection apparatus 50 are an example of the position control system of the embodiment of the present invention.

The position detection apparatus 50 according to the EXAMPLE 7 can be used as a position detection apparatus 50 also in EXAMPLE 2 ∼ 4.

By means of the position detection apparatus 50 according to the EXAMPLE 7, radiation is not required and a safety is improved.

### EXAMPLE 8

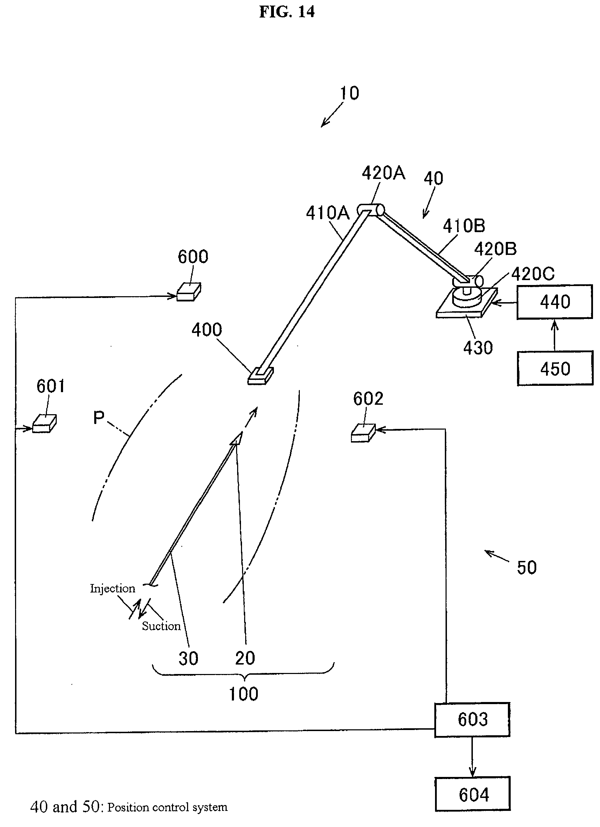

Figure 14 is a perspective view showing a configuration example of a schematic of an injection-suction system according to the EXAMPLE 8 of the embodiment of the present invention. The imaging unit and the position detection unit constitute the position detection apparatus 50 in the EXAMPLE I, but a measurement unit and a position detection unit constitute a position detection apparatus 50 in this embodiment. The measurement unit has at least three transmission distance measurement sensors capable of measuring a distance to the mark using a transmission method, and that can measure a three-dimensional position of a mark using the transmission distance measurement sensors. Radio rangefinders 600-602 constitute the transmission distance measurement sensors with the measurement unit of the position detection apparatus 50 in this embodiment.

A position detection apparatus 50 includes radio rangefinders 600-602 capable of measuring the fine head 20 also serving as a mark, a control unit 603 being capable of determining a position of the fine head 20, a display unit 604 that can display detected position of the fine head 20. The display unit 604 may be omitted if a computer automatically peforms confirming that the fine head 20 has reached a target position. The radio rangefinders 600-602 are an example of transmission distance measurement sensors which the measurement unit of the position detection apparatus has.

Each of the radio rangefinders 600-602 emit electromagnetic waves having different frequencies from each other toward the fine head 20, and obtain a distance from numbers of times of an oscillation until sensing electromagnetic waves having frequencies of which rangefinders respectively take charge, out of the electromagnetic waves reflected by the fine head 20. By knowing distances between the mark and three or more positions, and three-dimensional positions of the three or more positions, a three-dimensional position of the fine head 20 can be known because a three-dimensional position of the mark can be known by a calculation therefrom.

A frequency of the electromagnetic wave used to measure a distance by the measurement unit is preferably a frequency which is not easily absorbed by a human body.

The mark is preferably formed from a material which is easy to reflect an electromagnetic wave having a frequency to be used.

The ultra-fine pipe 30 is preferably formed from a material which is hard to reflect an electromagnetic wave having a frequency to be used.

For example, the radio rangefinders 600-602 is respectively fixed in predetermined positions separated from each other until a treatment is finished.

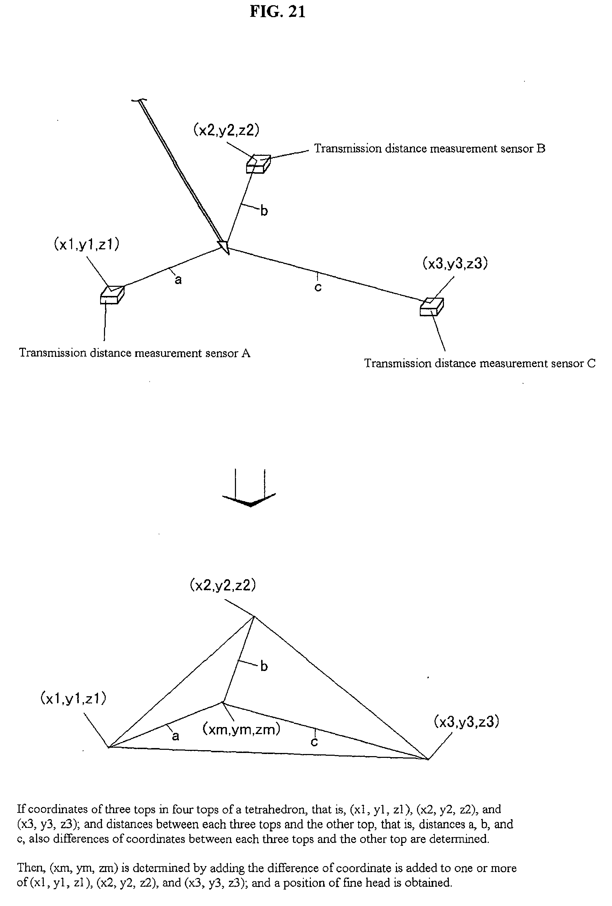

The control unit 603 is an example of a position detection unit. The control unit 603, for example, detects a position of the fine head 20 in the following procedure (see Figure 21):
1. Preparing the respective distances between the radio rangefinders 600-602 and the mark using the radio rangefinders 600-602;
2. Determining a difference in three-dimensional position between at least one of the radio rangefinders 600-602 and the mark by Pythagoras' theorem using the distances and each of positions of the radio rangefinders 600-602 which were prepared in 1; and
3. Obtaining the position of the head (20) by adding the three-dimensional positions of the radio rangefinders to the difference determined in 2.

Incidentally, the three-dimensional positions of at least one of the radio rangefinders 600-602 is measured in advance (before 3). When the radio rangefinders are fixed in a predetermined position, the measurement may be one time. Also, in 2, a position of the fine head 20 may be immediately determined using the distances and three-dimensional positions of the radio rangefinders 600-602 which were prepared in 1 when the three-dimensional positions of all the radio rangefinders 600-602 were measured.

Incidentally, the mark is better to be in a tip end side position of the fine head 20 or the ultra-fine pipe 30, and the mark need not be formed separately from the fine head 20 and the ultra-fine pipe 30. For example, it may be possible that a material which is easy to reflect an electromagnetic wave having a frequency to be used is applied to the fine head 20, and assuming that it is the mark.

Features of portions other than above portions are similar to the injection-suction system according to the EXAMPLE 1.

The movement apparatus 40 and the position detection apparatus 50 are an example of the position control system of the embodiment of the present invention.

The position detection apparatus 50 according to the EXAMPLE 8 can be used as a position detection apparatus 50 also in EXAMPLE 2 ∼ 4.

By means of the position detection apparatus 50 according to the EXAMPLE 8, radiation is not required and a safety is improved, and a maintenance is facilitated in many cases because moving parts are reduced by eliminating the arms.

### EXAMPLE 9

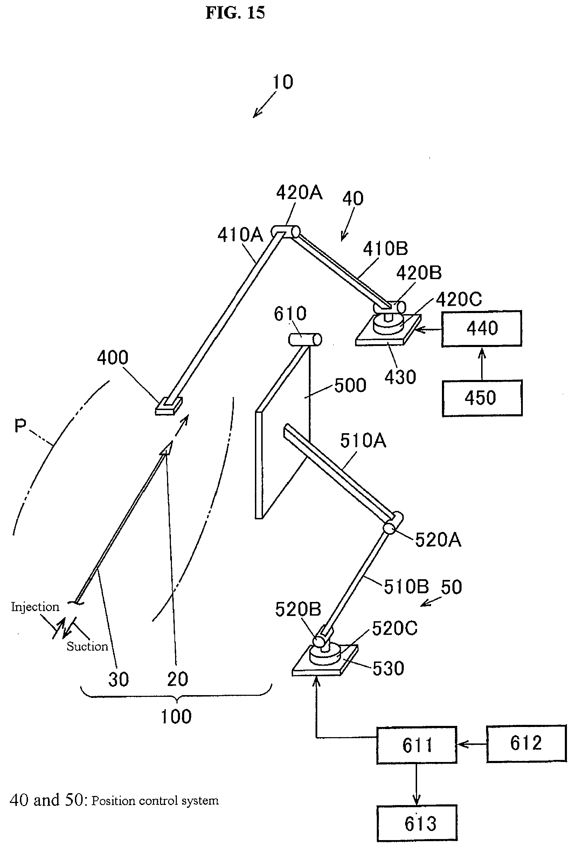

Figure 15 is a perspective view showing a configuration example of a schematic of an injection-suction system according to the EXAMPLE 5 of the embodiment of the present invention. The imaging unit and the position detection unit constitute the position detection apparatus 50 in the EXAMPLE 1, but an imaging unit and a measurement unit and a position detection unit constitute a position detection apparatus 50 in this embodiment.

The position detection apparatus 50 includes a radio rangefinder 610 capable of measuring the fine head 20 serving also a measurement unit mark. Also, the position detection apparatus 50 includes a X-ray CCD sensor 500 capable of detection and photographing radiation emitted from a mark of a radioactive substance which is applied to the fine head 20, first and second arms 510A and 510B for supporting the X-ray CCD sensor 500, first and second rotary drive units 520A and 520B capable of rotating movement of the first arm 510A and the second arms 510B respectively around a horizontal axis, a third rotary drive unit 520C capable of rotating movement of the second rotary drive unit 520B around a vertical axis, a base 530 which supports the third rotary drive unit 520C, a control unit 611 capable of driving and controlling each rotary drive units 587A, 587B, 587C and detecting a position of the fine head 20, an operation unit 612 that can indicate a three-dimensional position of the X-rays CCD sensor 500, a display unit 613 that can display detected position of the fine head 20. A position of forming an imaging unit mark of a radioactive substance may be also in a tip end side position of the ultra-fine pipe 30. The display unit 613 may be omitted if a computer automatically peforms confirming that the fine head 20 has reached a target position. The X-ray CCD sensor 500 is an example of a radiographic imaging sensor which an imaging unit of the position detection apparatus has. The radio rangefinder 610 is an example of a transmission distance measurement sensor with a measurement unit of the position detection apparatus.

In this embodiment, the mark is provided with two kinds of marks, an imaging unit mark and a measurement unit mark.

The imaging unit mark and the measurement unit mark are usually provided in the same position or are close position to each other. The imaging unit mark and the measurement unit mark may be the same one.

The imaging unit photographs the imaging unit mark by the X-ray CCD sensor 500. The measurement unit measures the measurement unit mark by the radio rangefinder 610. The position detection apparatus 50 determines a distance between the imaging unit mark and the X-ray CCD sensor 500, ie, a depth coordinate corresponding to a position of the imaging unit mark in image data photographed by the imaging unit, using a position where the imaging unit mark is projected in the X-ray CCD sensor 500, and a distance between the radio rangefinder 610 and the measurement unit mark. That is, the position detection apparatus 50 can obtain a three-dimensional position of the fine head 20 by determining positions of the imaging unit mark in two coordinate axes using the imaging unit, and determining a position of the imaging unit mark in remaining one coordinate axis using the measuring unit.

A pair of the X-ray CCD sensors are provided in the EXAMPLE 1, but one X-ray CCD sensor is provided in this embodiment.

A frequency of the electromagnetic wave used to measure a distance by the measurement unit is preferably a frequency which is not easily absorbed by a human body.

The fine head 20 is preferably formed from a material which is easy to reflect an electromagnetic wave having a frequency to be used.

The ultra-fine pipe 30 is preferably formed from a material which is hard to reflect an electromagnetic wave having a frequency to be used.

For example, the radio rangefinder 610 is fixed in a predetermined position which is on a side surface of the X-ray CCD sensor 500.

The operation unit 612 is configured to indicate a three-dimensional position to which the X-ray CCD sensor 500 moves by, for example, lever operation or computer.

The position detection apparatus 50 is configured so as to measure a three-dimensional position of a tip of the first arm 510A, that is, a three-dimensional position of the X-ray CCD sensor 500 precisely (eg, in micrometer units) by providing position sensors, angle sensors or the like on a joint portion between the first arm 510A and the second arm 510B, and a joint portion between the first arm 510B and the base 530, or providing a three-dimensional position sensor at a tip of the first arm 510A. The position sensors, the angle sensors, and the like provided on the joint portions, and the three-dimensional position sensor provided at the tip of the first arm 510A, are an example of a position detection unit for a radiographic imaging sensor capable of measuring a three-dimensional position of the radiographic imaging sensor with an imaging unit of the position detection apparatus.

The control unit 611 is an example of a position detection unit. The control unit 611 includes an image recognition processing unit capable of performing image recognition processing that can recognize images by a pattern recognition.

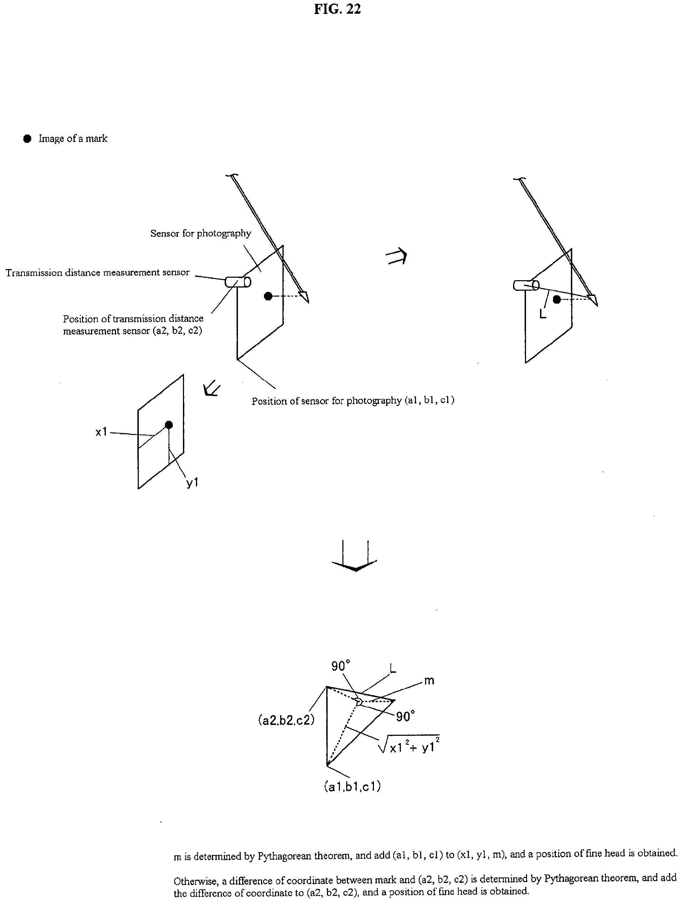

The control unit 611, for example, detects a position of the fine head 20 in the following procedure (see Figure 22):
1. Preparing image data using the X-ray CCD sensor 500. Also, preparing a distance between the radio rangefinder 610 and the measurement unit mark by the radio rangefinder 610;
2. In the image data, determining a position of the imaging unit mark within an imaging range of the image data by searching the image data for a portion corresponding to the imaging unit mark using the image recognition processing unit; and
3. Determining a distance between a position of the imaging unit mark and a position in which the imaging unit mark is projected on the X-ray CCD sensor 500 by Pythagoras' theorem using a distance between the radio rangefinder 610 and the measurement unit mark, a position of the imaging unit mark within an imaging range of the image data, a three-dimensional position of the X-ray CCD sensor 500, and a three-dimensional position of the radio rangefinder 610; and
4. Determining a three-dimensional position of the imaging unit mark within an imaging range of the image data by setting a distance between the position of the imaging unit mark and the position in which the imaging unit mark is projected on the X-ray CCD sensor 500 as a depth coordinate of a position of the imaging unit mark within an imaging range of the image data; and
5. Measuring, in advance, a three-dimensional position of the X-ray CCD sensor 500, and obtaining a position of the fine head by adding the three-dimensional position of the X-ray CCD sensor 500 to the three-dimensional position of the imaging unit mark within an imaging range of the image data.

A position of the imaging unit mark is regarded as the same as a position of the measurement unit mark because the imaging unit mark and the measurement unit mark are provided in substantially the same position in this embodiment. Incidentally, the three-dimensional position of the radio rangefinder 610 is measured in advance (before 3). When the radio rangefinder is fixed in a predetermined position, the measurement may be one time. When the radio rangefinder 610 is fixed in a predetermined position, a distance between a position of the radio rangefinder 610 and a position of the imaging unit mark within an imaging range of the image data can be determined by adding a position of the radio rangefinder 610 to a position of the imaging unit mark within an imaging range of the image data. In 3, a distance between a position of the imaging unit mark and a position in which the imaging unit mark is projected on the X-ray CCD sensor 500 is determined by Pythagoras' theorem treating a distance between a position of the radio rangefinder 610 and a position of the imaging unit mark within an imaging range of the image data as one side of two sides sandwiching a right angle; and treating a distance between the radio rangefinder 610 and the measurement unit mark i.e. a distance between the radio rangefinder 610 and the imaging unit mark as a hypotenuse.

Also, the following may be performed instead of 3-5:
3. Determining a difference in three-dimensional position between the measurement unit mark and the radio rangefinder 610 by Pythagoras' theorem using a distance between the radio rangefinder 610 and the measurement unit mark, a position of the imaging unit mark within an imaging range of the image data, a three-dimensional position of the X-ray CCD sensor 500, and a three-dimensional position of the radio rangefinder 610; and
4. Measuring, in advance, a three-dimensional position of the radio rangefinder 610, and obtaining a position of the fine head by adding the three-dimensional position of the radio rangefinder 610 to the difference in three-dimensional position determined in 3.

The control unit 611 moves the X-ray CCD sensor 500 to an indicated three-dimensional position based on a three-dimensional position indicated by the operation unit 550, by controlling the first to third rotary drive units 520A - 520C.

Incidentally, the imaging unit mark is better to be in a tip end side position of the fine head 20 or the ultra-fine pipe 30, and the imaging unit mark need not be formed separately from the fine head 20 and the ultra-fine pipe 30. In other words, it may be possible that a material of the fine head 20 is a substance capable of transmitting photographed by the X-ray CCD sensor 500, and the fine head 20 itself is treated as the imaging unit mark. For example, a radioactive substance may be kneaded to the material of the fine head 20. In addition, it may be possible that a material of the ultra-fine pipe 30 is a substance capable of transmitting photographed by the X-ray CCD sensor 500, and a total image or a wide range of image including a tip end side of the ultra-fine pipe 30 is projected in a photographed image data in the X-ray CCD sensor 500, and a portion corresponding to the tip end side in a image of the ultra-fine pipe 30 is treated as an image of the imaging unit mark in the image recognition processing. For example, it may be possible that the ultra-fine pipe 30 is made kneading a radioactive substance to a material of the ultra-fine pipe 30.

Also, the measurement unit mark is better to be in a tip end side position of the fine head 20 or the ultra-fine pipe 30, and the measurement unit mark may be formed separately from the fine head 20 and the ultra-fine pipe 30. For example, it may be possible that a material which is easy to reflect an electromagnetic wave having a frequency to be used is applied to the fine head 20, and that is treated as the mark.

A range which is marked by doctor hand or the like is in a range of accuracy of millimeters at most though it varies depending on a manual dexterity of the doctor corresponding to the work. In addition, the fine head 20 is just moved to 'generally' near center of the range in many cases. Therefore, it is possible to use large X-ray CCD sensor for digital X-ray imaging which are common that accuracy is low to reduce a cost because they are so huge as the position detection apparatus 50. It is also possible to fix the X-ray CCD sensor in a predetermined position without using the arms until a treatment is finished when using large X-ray CCD sensor for digital X-ray imaging as the position detection apparatus 50.

A maintenance is facilitated in many cases because moving parts are reduced by eliminating the arms. In the case the fine head 20 is desired to move inside or outside of a cell membrane certainly, a movement of the fine head 20 with high accuracy is required, but an accuracy of a position detection may be also lower since it is possible to determine whether the tip end of the ultra-fine pipe 30 is inside or outside of a cell membrane by a method such as measuring a pressure by attaching a pressure gauge to the pump. Therefore, it is possible to use the large X-ray CCD sensor for digital X-ray imaging as the position detection apparatus 50 even in this case.

The position detection apparatus 50 may be provided with a rotary drive unit also in between the first arm 510A and the X-ray CCD sensor 500.

In addition, the position detection apparatus 50 may be one that the first arm 510A and the first rotary drive unit 520A are omitted and the second arm 510B is an elastic arm.

Moreover, it is also possible to use an X-ray CMOS sensor in place of the X-ray CCD sensor.

Features of portions other than above portions are similar to the injection-suction system according to the EXAMPLE 1.

The movement apparatus 40 and the position detection apparatus 50 are an example of the position control system of the embodiment of the present invention.

The position detection apparatus 50 according to the EXAMPLE 9 can be used as a position detection apparatus 50 also in EXAMPLE 2 ∼ 4.

### EXAMPLE 10

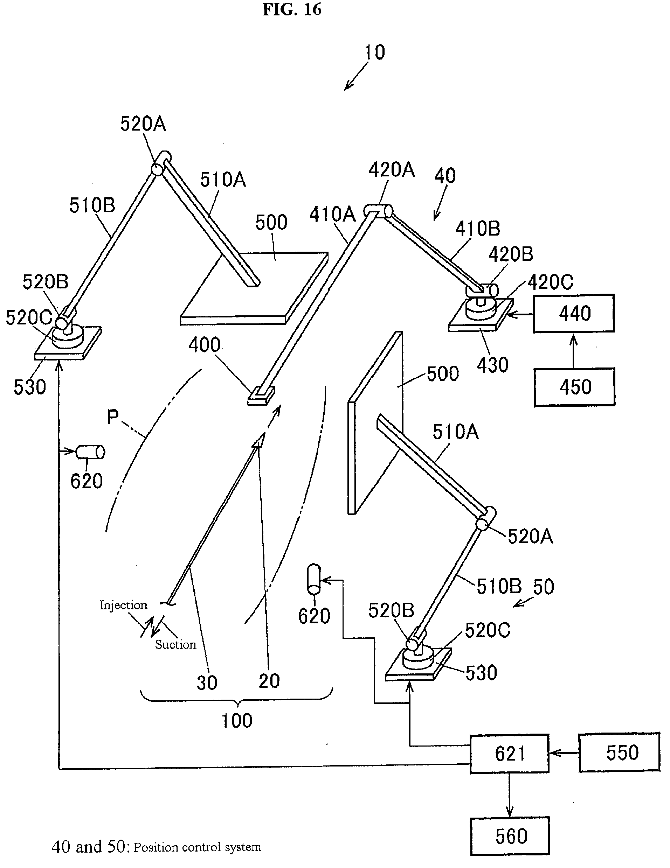

Figure 16 is a perspective view showing a configuration example of a schematic of an injection-suction system according to the EXAMPLE 10 of the embodiment of the present invention. Although radiation emitted from a mark of a radioactive substance has been detected and photographed by the X-ray CCD sensors 500 in the EXAMPLE 1, but a substance which can absorb a radioactive substance easily i.e. a substance which is hard to be projected on the X-ray CCD sensors 500 constitutes the mark and radiation is detected and photographed while irradiating radiation for the mark by irradiation units 620 in this embodiment. That is, detection and photographing in the EXAMPLE 1 is positive type, but detection and photographing in the EXAMPLE 10 is negative type.

A position detection apparatus 50 includes irradiation units 620 which can irradiate radiation for a mark, a pair of X-ray CCD sensors 500 capable of detection and photographing radiation emitted from the irradiation units 620, first and second arms 510A and 510B for supporting the X-ray CCD sensors 500, first and second rotary drive units 520A and 520B capable of rotating movement of the first arms 510A and the second arms 510B respectively around a horizontal axis, third rotary drive units 520C capable of rotating movement of the second rotary drive units 520B around a vertical axis, bases 530 which support the third rotary drive units 520C, a control unit 621 capable of driving and controlling each rotary drive units 587A, 587B, 587C and detecting a position of the fine head 20, an operation unit 550 that can indicate three-dimensional positions of the X-rays CCD sensors 500, a display unit 560 that can display detected position of the fine head 20. A position of forming the mark of a substance which can absorb a radioactive substance easily may be also in a tip end side position of the ultra-fine pipe 30. The display unit 560 may be omitted if a computer automatically peforms confirming that the fine head 20 has reached a target position. The X-ray CCD sensors 500 are an example of a radiographic imaging sensor which an imaging unit of the position detection apparatus has.

By detecting positions of the mark in a direction perpendicular to each other by the pair of X-ray CCD sensors 500, a three-dimensional position of the fine head 20 can be known.

The operation unit 550 is configured to indicate three-dimensional positions to which the X-ray CCD sensors 500 move by, for example, lever operation or computer.

The position detection apparatus 50 is configured so as to measure three-dimensional positions of tips of the first arms 510A, that is, three-dimensional positions of the X-ray CCD sensors 500 precisely (eg, in micrometer units) by providing position sensors, angle sensors or the like on joint portions between the first arms 510A and the second arms 510B, and joint portions between the first arms 510B and the bases 530, or providing three-dimensional position sensors at tips of the first arms 510A. The position sensors, the angle sensors, and the like provided on the joint portions, and the three-dimensional position sensors provided at the tips of the first arms 510A, are an example of a position detection unit for a radiographic imaging sensor capable of measuring a three-dimensional position of the radiographic imaging sensor with an imaging unit of the position detection apparatus.

The control unit 621 is an example of a position detection unit. The control unit 621 includes an image recognition processing unit capable of performing image recognition processing that can recognize images by a pattern recognition.

The control unit 621, for example, detects a position of the fine head 20 in the following procedure (see Figure 19):
1. Preparing a pair of image data photographed from a direction perpendicular to each other using the pair of X-ray CCD sensors 500. Incidentally, the image data is photographed while irradiating radiation for the mark by the irradiation units 620;
2. In each of the image data, determining a two-dimensional position of a mark within an imaging range of the image data by searching the image data for a portion corresponding to the mark using the image recognition processing unit. Then, determining a three-dimensional position of the mark in the imaging range of an image data by synthesizing two-dimensional positions of the mark in a pair of image data in an orthogonal direction; and
3. Adding three-dimensional positions of the X-ray CCD sensors 500 to the three-dimensional position of the mark in the imaging range of an image data, and obtaining a position of the fine head 20.

Also, the control unit 621, based on three-dimensional positions indicated by the operation unit 550, by controlling the first to third rotation drive units 520A ∼ 520C, moves the X-ray CCD sensors 500 to indicated three-dimensional positions.

Incidentally, the mark is better to be in a tip end side position of the fine head 20 or the ultra-fine pipe 30, and the mark need not be formed separately from the fine head 20 and the ultra-fine pipe 30. In other words, it may be possible that a material of the fine head 20 is a substance capable of transmitting photographed by the X-ray CCD sensors 500, and the fine head 20 itself is treated as the mark. For example, a radioactive substance may be kneaded to the material of the fine head 20. In addition, it may be possible that a material of the ultra-fine pipe 30 is a substance capable of transmitting photographed by the X-ray CCD sensors 500, and a total image or a wide range of image including a tip end side of the ultra-fine pipe 30 is projected in a photographed image data in the X-ray CCD sensors 500, and a portion corresponding to the tip end side in a image of the ultra-fine pipe 30 is treated as an image of the mark in the image recognition processing. For example, it may be possible that the ultra-fine pipe 30 is made kneading a radioactive substance to a material of the ultra-fine pipe 30.

A range which is marked by doctor hand or the like is in a range of accuracy of millimeters at most though it varies depending on a manual dexterity of the doctor corresponding to the work. In addition, the fine head 20 is just moved to 'generally' near center of the range in many cases. Therefore, it is possible to use large X-ray CCD sensors for digital X-ray imaging which are common that accuracy is low to reduce a cost because they are so huge as the position detection apparatus 50. It is also possible to fix the X-ray CCD sensors in a predetermined position without using the arms until a treatment is finished when using large X-ray CCD sensors for digital X-ray imaging as the position detection apparatus 50. A maintenance is facilitated in many cases because moving parts are reduced by eliminating the arms. If the fine head 20 is desired to move inside or outside of a cell membrane certainly, a movement of the fine head 20 with high accuracy is required. But an accuracy of a position detection may be also lower since it is possible to determine whether the tip end of the ultra-fine pipe 30 is inside or outside of a cell membrane by a method such as measuring a pressure by attaching a pressure gauge to the pump. Therefore, it is possible to use the large X-ray CCD sensors for digital X-ray imaging as the position detection apparatus 50 even in this case.

The position detection apparatus 50 may be provided with a rotary drive units also in between the first arms 510A and the X-ray CCD sensors 500.

In addition, the position detection apparatus 50 may be one that the first arms 510A and the first rotary drive units 520A are omitted and the second arms 510B are elastic arms.

Moreover, it is also possible to use X-ray CMOS sensors in place of the X-ray CCD sensors.

Features of portions other than above portions are similar to the injection-suction system according to the EXAMPLE 1.

The movement apparatus 40 and the position detection apparatus 50 are an example of the position control system of the embodiment of the present invention.

The position detection apparatus 50 according to the EXAMPLE 10 can be used as a position detection apparatus 50 also in EXAMPLE 2 ∼ 4.

### EXAMPLE 11

Formed of a magnetic material, a head that can be moved by a magnetic field in the body, it is attached to the head in a state where the leading end is opened, injecting and with a, and injection or possible suction pipe fluid through an opening portion in the distal end suction apparatus.

Such as ultra-fine pipe 30 in the embodiment of the 1-3, it corresponds to the embodiment.

### EXAMPLE 12

Formed of a magnetic material, the maximum width is 100µm or less, a head that can be moved by a magnetic field in the body, attached to the head in a state where the leading end is opened, it can be injected or aspirated liquids through the opening of the tip injection-suction apparatus provided with, and the pipe.

This embodiment is obtained by limiting the diameter of the pipes primarily in the eleventh embodiment.

### EXAMPLE 13

Formed of a magnetic material, the maximum width is 5µm or less, a head that can be moved by a magnetic field in the body, attached to the head in a state where the leading end is opened, it can be injected or aspirated liquids through the opening of the tip injection-suction apparatus provided with, and the pipe.

This embodiment is obtained by limiting the diameter of the pipes primarily in the eleventh embodiment.

### EXAMPLE 14

Formed of a magnetic material, the maximum width is 1µm or less, a head that can be moved by a magnetic field in the body, attached to the head in a state where the leading end is opened, it can be injected or aspirated liquids through the opening of the tip injection-suction apparatus provided with, and the pipe.

This embodiment is obtained by limiting the diameter of the pipes primarily in the eleventh embodiment.

### EXAMPLE 15

The pipe, the injection-suction apparatus according to the eleventh to fourteenth embodiments of which are formed of a nonmagnetic material.

This embodiment is obtained by limiting the material of the pipes primarily in the embodiment of the 1 to 14.

### EXAMPLE 16

The head tip injection-suction apparatus according to any one of the embodiments of the 11 to 15 which is dissected capable to form a living tissue.

This embodiment is obtained by limiting the like shape of the head tip primarily in the embodiment of the 11 to 15.

### EXAMPLE 17

The head includes a first head portion having a narrowed shape toward the tip, are arranged said first head section by a predetermined distance, the second having a narrowed shape toward the rear end a head portion, said first and second and a connecting portion connecting the head portion, the pipe 11 with its tip is provided so as to be positioned between said first and second head portions The injection-suction apparatus according to any one of to 16 embodiments.

This embodiment is obtained by limiting the like overall shape of the head mainly in the embodiment of the 11 to 16.

### EXAMPLE 18

The pipe tip injection-suction apparatus according to any one of the embodiments of the 11 to 17 that is dissected capable to form a living tissue.

This embodiment is obtained by limiting the like shape of the pipe tip primarily in the embodiment of the 11 to 17.

### EXAMPLE 19

Is formed of a magnetic material, and has an electromagnet and capable of imparting a magnetic field to the head that can be moved by a magnetic field in the body, and a controllable movement control unit and the magnitude and direction of the magnetic force exerted on the head by the magnetic field the electromagnet grants a movement apparatus is attached to the head in a state where the head or tip is open, to determine the location of the marks present on the distal end side position of the injection or aspiration pipe fluid through an opening in the distal end, the Position control system with a detectable position detection apparatus a position of the head based on the position of the mark.

Such embodiments of the first to 10 are configured by using the present embodiment.

### EXAMPLE 20

Formed of a magnetic material, is the maximum width of 100µm or less, the electromagnet and capable of imparting a magnetic field on the head that can be moved by a magnetic field in the body, and controlling the magnitude and direction of the magnetic force exerted on the head by the magnetic field the electromagnet grants The presence and movement apparatus having a possible movement control section, attached to the head in a state where the head or tip is open, the front end position of the infusion or aspiration pipe with liquid through the opening of the distal end Position control system with to determine the position of the mark, and a detectable position detection apparatus a position of the head based on the position of the mark.

This embodiment is obtained by limiting the diameter of the pipes primarily in the nineteenth embodiment.

### EXAMPLE 21

Formed of a magnetic material, is the maximum width of 5µm or less, the electromagnet and capable of imparting a magnetic field on the head that can be moved by a magnetic field in the body, and controlling the magnitude and direction of the magnetic force exerted on the head by the magnetic field the electromagnet grants The presence and movement apparatus having a possible movement control section, attached to the head in a state where the head or tip is open, the front end position of the infusion or aspiration pipe with liquid through the opening of the distal end Position control system with to determine the position of the mark, and a detectable position detection apparatus a position of the head based on the position of the mark.

This embodiment is obtained by limiting the diameter of the pipes primarily in the nineteenth embodiment.

### EXAMPLE 22

Formed of a magnetic material, is the maximum width of 1µm or less, the electromagnet and capable of imparting a magnetic field on the head that can be moved by a magnetic field in the body, and controlling the magnitude and direction of the magnetic force exerted on the head by the magnetic field the electromagnet grants The presence and movement apparatus having a possible movement control section, attached to the head in a state where the head or tip is open, the front end position of the infusion or aspiration pipe with liquid through the opening of the distal end Position control system with to determine the position of the mark, and a detectable position detection apparatus a position of the head based on the position of the mark.

This embodiment is obtained by limiting the diameter of the pipes primarily in the nineteenth embodiment.

### EXAMPLE 23

Movement control unit of the mobile apparatus, by adjusting the magnitude and direction of the magnetic force exerted on the head by the magnetic field of the electromagnet is granted, the pipe of the head as plugged substantially straight with respect to the target position The position control system according to an embodiment of the 19 to 22 and a movement control unit capable of performing the traveling direction control.

This embodiment, in the embodiment of the 19 to 22, is obtained by limiting the operation of the movement control part of the main movement apparatus.

### EXAMPLE 24

The position control system according to the embodiment of the 19 to 22, characterized in that the position detection apparatus includes:
an imaging unit having a radiographic imaging sensor and capable of capturing an image of the mark using the radiographic imaging sensor; and
a position detection unit capable of detecting the position of the head on the basis of the image of the mark captured by the imaging unit.

The position detection apparatus includes an imaging section capable of photographing the mark by the fluoroscopic imaging sensor includes a fluoroscopic imaging sensor, capable of detecting the position the position of the head on the basis of the mark photographed by the photographing section a position detection apparatus having a detecting portion, and a position control system according to.

The 1-7 and the tenth embodiment is configured by using the present embodiment.

### EXAMPLE 25

Movement control unit of the mobile apparatus, by adjusting the magnitude and direction of the magnetic force exerted on the head by the magnetic field of the electromagnet is granted, the pipe of the head as plugged substantially straight with respect to the target position The position control system according to the 24th embodiment of the movement control unit capable of performing the traveling direction control.

This embodiment, in the 24 embodiment, is obtained by limiting the operation of the movement control part of the main movement apparatus.

### EXAMPLE 26

The distal end side position of the head or the pipe, wherein the head and the pipe to form a separately the mark, the position control system according to an embodiment of the 24 or 25.

This embodiment, in the embodiment of the 24 or 25 is obtained mainly limit the formation position of the mark.

### EXAMPLE 27

The material of the head is capable of transmitting captured material by the fluoroscopic imaging sensor, the 24th or the 25th position control system according to the embodiment of.

This embodiment, in the first 24 or second 25 embodiment, is obtained by primarily limit the formation position of the mark.

### EXAMPLE 28

Position detecting section of the position detection apparatus has an image recognition processing unit capable of performing an image recognition process, the following 1 to 3 at the position detector capable of detecting the position of the head in step There, the position control system according to any one of the embodiments of the 24 to 27.
1. Prepare the image data by the imaging unit.
2. In the image data to locate the portion corresponding to the mark by the image recognition processing unit to determine the position of the mark in the imaging range of the image data.
3. Previously it said it is also possible to measure the three-dimensional position of the fluoroscopic imaging sensor imaging unit has, at the position of the mark in the imaging range of the image data, in addition to three-dimensional position of the fluoroscopic imaging sensor with said imaging unit, It determines the position of the head.

This embodiment, in the embodiment of the 24 to 27, is obtained by primarily limit the procedure of position detection of the head.

This embodiment includes a three-dimensional position of the fluoroscopic imaging sensor having a photographing unit, also the embodiment in which it is better that be measured by such pre-human hands, such as during manufacture of the imaging unit. For example, when performing imaging by using a giant CCD sensor, usually, it does not move by securing a large CCD sensor in position, and corresponds to the present embodiment.

### EXAMPLE 29

Perspective the position detection apparatus, the imaging unit is a three-dimensional position of the fluoroscopic imaging sensor having a measurable fluoroscopic imaging sensor position detecting section having, by the fluoroscopic imaging sensor position detecting unit, with said imaging unit and a position detector capable of measuring the three-dimensional position of the imaging sensor, 28 and a position control system according to the embodiment of.

This embodiment, in the 28 embodiment of the three-dimensional position of the fluoroscopic imaging sensor having a photographing unit, instead of measuring the like beforehand human hand, such as when measuring the dedicated position sensor in the form of implementation.

### EXAMPLE 30

Fluoroscopic imaging sensor included in the imaging section is a pair of two-dimensional image sensor, the image data to be prepared in 1, a pair of image data taken from a direction perpendicular to each other, in two, each of the image The portion corresponding to the mark in the data, looking through the image recognition processing unit obtains a two-dimensional position of the mark in the imaging range of the image data, then, two-dimensional of the mark in the imaging range of the pair of the image data position is synthesized and a in the orthogonal direction to obtain a three-dimensional position of the mark in the imaging range of the image data, the 3, it is also possible to measure the three-dimensional position of the fluoroscopic imaging sensor having beforehand the imaging unit, and the image data the three-dimensional position of the mark in the imaging range, wherein in addition to three-dimensional position of the fluoroscopic imaging sensor imaging unit has, to the position of the head, and position control according to the 28th or the 29th embodiment of the system.

This embodiment, in the first 28 or second 29 embodiment, is obtained by limiting the steps of 1-3.

Instead of 2-3,
The portion corresponding to the mark in each of the image data by searching through the image recognition processing unit obtains a two-dimensional position of the mark in the imaging range of the image data,

Then, the two-dimensional position of the mark in the imaging range of each of the image data, in addition to three-dimensional position of the fluoroscopic imaging sensor obtains the three-dimensional position of the mark in the imaging range of each of said image data synthesizes the three-dimensional position of the mark in the imaging range of the pair of the image data in the orthogonal direction to the position of the head.

It may be.

First to fourth and tenth embodiment is configured by using the present embodiment.

### EXAMPLE 31

The fluoroscopic imaging sensor imaging unit has is a two-dimensional image sensor for obtaining cross-sectional images, the image data to be prepared in 1 is image data of the face horizontal multiple of each other in the imaging target, in two, Locate the portion corresponding to the mark in the image data by the image recognition processing unit obtains a two-dimensional position of the mark in the imaging range of the image data, the three, three-dimensional fluoroscopic imaging sensor with said imaging unit The position, to obtain a three-dimensional position of the reference position of the surface of the imaging target in the fluoroscopic imaging sensor with said imaging unit, 28 is a position control system according to the embodiment of.

This embodiment, in the 28 embodiment, is obtained by limiting the steps of 1-3.

Including fifth to seventh embodiment is configured by using the present embodiment.

### EXAMPLE 32

The photographing unit fluoroscopic imaging sensor having a is a two-dimensional image sensor for obtaining cross-sectional images, the fluoroscopic imaging sensor position detecting unit, measuring the reference position of the range of the imaging target in the fluoroscopic imaging sensor with said imaging unit is a possible position detection unit, the image data to be prepared in 1 is image data of the face horizontal multiple of each other in the imaging target, in two, a portion corresponding to the mark in the image data the image Locate the recognition processing unit obtains a two-dimensional position of the mark in the imaging range of the image data, the three, the three-dimensional position of the fluoroscopic imaging sensor with said imaging unit, fluoroscopic imaging sensor with said imaging unit to obtain a three-dimensional position of the reference position of the surface of the imaging object, 29 is a position control system according to the embodiment of.

This embodiment, in the 29th embodiment, is obtained by limiting the steps of 1-3.

This embodiment, in the 31 embodiment of the three-dimensional position of the fluoroscopic imaging sensor having a photographing unit, instead of measuring the like beforehand human hand, such as when measuring the dedicated position sensor in the form of implementation.

### EXAMPLE 33

Position detecting section of the position detection apparatus has an image recognition processing unit capable of performing an image recognition process, the material of the pipe is a material capable of transmitting photographed by the fluoroscopic imaging sensor, the position detector image recognition processing unit with the position detection unit is in the image recognition processing unit that handles the portion corresponding to the distal end side of the pipe as the image of the mark of said pipe image of reflected in image data prepared by said imaging unit There, the first 24 or 25 and position control system according to an embodiment of the.

This embodiment, in the first 24 or second 25 embodiment, is obtained by primarily limit the formation position of the mark.

### EXAMPLE 34

Position detecting section of the position detection apparatus has an image recognition processing unit capable of performing an image recognition process, the following 1 to 3 at the position detector capable of detecting the position of the head in step there, 33 and position control system according to an embodiment of the.
1. Prepare the image data by the imaging unit.
2. In the image data to locate the portion corresponding to the mark by the image recognition processing unit to determine the position of the mark in the imaging range of the image data.
3. Previously it said it is also possible to measure the three-dimensional position of the fluoroscopic imaging sensor imaging unit has, at the position of the mark in the imaging range of the image data, in addition to three-dimensional position of the fluoroscopic imaging sensor with said imaging unit, It determines the position of the head.

This embodiment, in the 33 embodiment, is obtained by primarily limiting the procedure for position detection of the head.

This embodiment includes a three-dimensional position of the fluoroscopic imaging sensor having a photographing unit, also the embodiment in which it is better that be measured by such pre-human hands, such as during manufacture of the imaging unit. For example, when performing imaging by using a giant CCD sensor, usually, it does not move by securing a large CCD sensor in position, and corresponds to the present embodiment.

### EXAMPLE 35

Position detecting section of the position detector is a position detector capable of detecting the position of the head in the following 1-3 step, the 33 position control system according to the embodiment of.
1. Prepare the image data by the imaging unit.
2. Address the portion corresponding to the distal end side of the pipe of the image of the pipe reflected in the image data as an image of the mark, find the portion corresponding to the mark in the image data by the image recognition processing unit, wherein It determines the position of the mark in the imaging range of the image data.
3. Previously it said it is also possible to measure the three-dimensional position of the fluoroscopic imaging sensor imaging unit has, at the position of the mark in the imaging range of the image data, in addition to three-dimensional position of the fluoroscopic imaging sensor with said imaging unit, It determines the position of the head.

This embodiment, the second 33 embodiment, is obtained by primarily limiting the procedure for position detection of the head.

This embodiment includes a three-dimensional position of the fluoroscopic imaging sensor having a photographing unit, also the embodiment in which it is better that be measured by such pre-human hands, such as during manufacture of the imaging unit. For example, when performing imaging by using a giant CCD sensor, usually, it does not move by securing a large CCD sensor in position, and corresponds to the present embodiment.

### EXAMPLE 36

Perspective the position detection apparatus, the imaging unit is a three-dimensional position of the fluoroscopic imaging sensor having a measurable fluoroscopic imaging sensor position detecting section having, by the fluoroscopic imaging sensor position detecting unit, with said imaging unit and a position detector capable of measuring the three-dimensional position of the imaging sensor, the position control system according to an embodiment of the 34 or 35.

This embodiment, an embodiment of the 34 or 35, the three-dimensional position of the fluoroscopic imaging sensor having a photographing unit, instead of measuring the like in advance by a human hand when it is measured by a dedicated position sensor It is the embodiment of such.

### EXAMPLE 37

Fluoroscopic imaging sensor included in the imaging section is a pair of two-dimensional image sensor, the image data to be prepared in 1, a pair of image data taken from a direction perpendicular to each other, in two, each of the image The portion corresponding to the mark in the data, looking through the image recognition processing unit obtains a two-dimensional position of the mark in the imaging range of the image data, then, two-dimensional of the mark in the imaging range of the pair of the image data position is synthesized and a in the orthogonal direction to obtain a three-dimensional position of the mark in the imaging range of the image data, the 3, it is also possible to measure the three-dimensional position of the fluoroscopic imaging sensor having beforehand the imaging unit, and the image data the three-dimensional position of the mark in the imaging range, to the addition of three-dimensional position of the fluoroscopic imaging sensor with said imaging unit, and the position of the head, one embodiment of the 34 to 36 Position control system according.

This embodiment, in the embodiment of the 34 to 36, is obtained by limiting the steps of 1-3.

Instead of 2-3,
The portion corresponding to the mark in each of the image data by searching through the image recognition processing unit obtains a two-dimensional position of the mark in the imaging range of the image data,

Then, the two-dimensional position of the mark in the imaging range of each of the image data, in addition to three-dimensional position of the fluoroscopic imaging sensor obtains the three-dimensional position of the mark in the imaging range of each of said image data synthesizes the three-dimensional position of the mark in the imaging range of the pair of the image data in the orthogonal direction to the position of the head.

It may be.

The 1-4, in the tenth embodiment, like in the case of handling as a mark the front end side of the image of the entire image or a wide range of the pipe, this embodiment is used.

### EXAMPLE 38

The fluoroscopic imaging sensor imaging unit has is a two-dimensional image sensor for obtaining cross-sectional images, the image data to be prepared in 1 is image data of the face horizontal multiple of each other in the imaging target, in two, Locate the portion corresponding to the pipe in the image data by the image recognition processing unit, and found in the image data obtained by photographing the most outside end of the person who is not a root side of the pipe of the portion corresponding to the pipe to be found were are dealing a portion corresponding to the mark one obtains a two-dimensional position of said mark in said image data, at 3, and the perspective of the three-dimensional position of the fluoroscopic imaging sensor imaging unit having the imaging unit is better to obtain a three-dimensional position of the reference position of the surface of the imaging target of the imaging sensor, the position control system according to any one of the embodiments of the 34 to 36.

This embodiment, in the embodiment of the 34 to 36, is obtained by limiting the steps of 1-3.

In the embodiment of the 5-7, such as in the case of handling as a mark the front end side of the image of the entire image or a wide range of the pipe, this embodiment is used.

### EXAMPLE 39

The photographing unit fluoroscopic imaging sensor having a is a two-dimensional image sensor for obtaining cross-sectional images, the fluoroscopic imaging sensor position detecting unit, measuring the reference position of the range of the imaging target in the fluoroscopic imaging sensor with said imaging unit is a possible position detection unit, the image data to be prepared in 1 is image data of the face horizontal multiple of each other in the imaging target, in two, a portion corresponding to the pipe in the image data the image Locate the recognition processing unit, and dealing with the found the pipe base side at the portion corresponding to the mark to those found in the image data obtained by photographing the most outside end of the person is not within the portion corresponding to the pipe, to obtain a two-dimensional position of said mark in said image data, at 3, wherein the three-dimensional position of the fluoroscopic imaging sensor imaging unit has, tertiary reference position of the surface of the imaging target of the fluoroscopic imaging sensor with said imaging unit It was the original position, the 36 position control system according to an embodiment of the.

This embodiment, in the 36th embodiment, is obtained by limiting the steps of 1-3.

This embodiment, in the 38th embodiment, the three-dimensional position of the fluoroscopic imaging sensor having a photographing unit, instead of measuring the like beforehand human hand, such as when measuring the dedicated position sensor in the form of implementation.

### EXAMPLE 40

The position detection apparatus, the surface of each imaging object is to interpolate between the horizontal plurality of cross-sectional image data to each other and a cross-sectional image three-dimensional processing section for producing a three-dimensional image data, a said imaging unit fluoroscopic imaging sensor is a two-dimensional image sensor for obtaining cross-sectional images, the image recognition processing unit, and an image recognition processing unit capable of performing three-dimensional image recognition processing, in one cross-sectional image three-dimensional by processing unit, the imaging unit each of the imaging target surface taken by the fluoroscopic imaging sensor is provided with three-dimensional image data produced from the horizontal of a plurality of image data together with, in two, and the image data said relevant part mark searching by the image recognition processing unit obtains the three-dimensional position of the mark in the imaging range of the image data, the three, the three-dimensional position of the fluoroscopic imaging sensor with said imaging unit in, The position control system according to any one of the embodiments of the is set to three-dimensional position of the reference position range of the imaging target in the fluoroscopic imaging sensor imaging unit has, 34 to 36.

This embodiment, in the embodiment of the 34 to 36, is obtained by limiting the steps of 1-3.

In the embodiment of the 5-7, such as in the case of handling as a mark the front end side of the image of the entire image or a wide range of the pipe, this embodiment is used.

### EXAMPLE 41

The position detection apparatus, the surface of each imaging object is to interpolate between the horizontal plurality of cross-sectional image data to each other and a cross-sectional image three-dimensional processing section for producing a three-dimensional image data, a said imaging unit fluoroscopic imaging sensor is a two-dimensional image sensor for obtaining cross-sectional images, the image recognition processing unit, and an image recognition processing unit capable of performing three-dimensional image recognition processing, the fluoroscopic imaging sensor position detection parts is a measurable position detector to a reference position range of the imaging target in the fluoroscopic imaging sensor with said imaging unit, at 1, the cross-sectional image three-dimensional processing section, a fluoroscopy with said imaging unit Prepare a three-dimensional image data generated from the image data of the face horizontal plurality of mutually respective photographic subject is captured by the sensor, the two said portions corresponding to the mark in the image data the image recognition processing unit to determine the three dimensional position of the mark in the imaging range of the image data searching by at 3, the three-dimensional position of the fluoroscopic imaging sensor with said imaging unit, the imaging target of the fluoroscopic imaging sensor with said imaging unit to obtain a three-dimensional position of the range of the reference position, the second 36 and the position control system according to any one of the embodiment.

This embodiment, in the 36th embodiment, is obtained by limiting the steps of 1-3.

This embodiment, in the 40 embodiment of the three-dimensional position of the fluoroscopic imaging sensor having a photographing unit, instead of measuring the like beforehand human hand, such as when measuring the dedicated position sensor in the form of implementation.

### EXAMPLE 42

The position detection apparatus has a transmission distance measuring sensor distance possible measurement with transparently the mark 3 above, wherein the transmission distance measuring sensor can measure the distance between the mark and the transmission distance measuring sensor Na and measuring unit, on the basis of the distance is a position detection apparatus having a detectable position detector the position of the head, the position control system according to an embodiment of the 19 to 22.

Such as the eighth embodiment is configured using the present embodiment.

### EXAMPLE 43

Movement control unit of the mobile apparatus, by adjusting the magnitude and direction of the magnetic force exerted on the head by the magnetic field of the electromagnet is granted, the pipe of the head as plugged substantially straight with respect to the target position 42 and the position control system according to an embodiment of the movement control unit capable of performing the traveling direction control.

This embodiment, in the 42 embodiment, is obtained by limiting the operation of the movement control part of the main movement apparatus.

### EXAMPLE 44

The distal end side position of the head or the pipe, the head and the pipe and to form a separately the mark, the position control system according to an embodiment of the 42 or 43.

This embodiment, in the embodiment of the 42 or 43 is obtained mainly limit the formation position of the mark.

### EXAMPLE 45

The material of the head is a substance capable of transmitting the ranging by the transmission distance measuring sensor, the position control system according to an embodiment of the 42 or 43.

This embodiment, in the embodiment of the 42 or 43 is obtained mainly limit the formation position of the mark.

### EXAMPLE 46

Position detecting section of the position detector is a position detector capable of detecting the position of the head in the following 1-3 step, according to any one of the first 42 to 45 the embodiment of position control system.
1. By the transmission distance measurement sensor, we are prepared and each of the transmission distance measuring sensor for the distance between the mark.
2. Pre is previously measured three-dimensional position of said transmission distance measuring sensor, the Pythagorean theorem, using a three-dimensional position of said transmission distance measuring sensor and the distance to one or more of the transmission distance measuring sensor I ask the difference between the three-dimensional position of said mark.

The difference obtained in 3.2, in addition to three-dimensional position of said transmission distance measuring sensor, and the position of the head.

This embodiment, in the embodiment of 42 to 45, is obtained by primarily limiting the procedure for position detection of the head.

This embodiment, the three-dimensional position of the perspective distance measurement sensor having a measuring unit, also the embodiment in which it is better that be measured by such pre-human hands, such as during manufacture of the measuring unit contains. For example, when performing a measurement using an electromagnetic wave distance measuring sensor, usually, it does not move by an electromagnetic wave distance measuring sensor fixed to a predetermined position, and corresponds to the present embodiment.

### EXAMPLE 47

The position detection apparatus, the three-dimensional position of the transmission distance measurement sensor has a measurement possible transmission distance measuring sensor position detection unit, by the transmission distance measuring sensor position detection unit, of the transmission distance measuring sensor and a position detector capable of measuring the three-dimensional positions, the position control system according to the 46th embodiment.

This embodiment, in the 46th embodiment, the three-dimensional position of the transmission distance measuring sensor having a measuring unit, rather than measures such as the pre-human hands, such as when it is measured by a dedicated position sensor It is the embodiment of the present invention.

### EXAMPLE 48

The mark is a mark for the mark and the measurement unit for photographing unit, the position detection apparatus includes an imaging section capable of photographing a mark for the imaging unit by the fluoroscopic imaging sensor includes a fluoroscopic imaging sensor, transparent the distance and the possible measurement measuring section between the mark for the measurement unit and the transmission distance measuring sensor by the transmission distance measurement sensor has a transmission distance measuring sensor that distance can be measured between the mark for measuring unit in, and a position detection apparatus having a detectable position detector the position of the head on the basis of the photographing unit marks and the distance taken by the imaging unit, and the position according to the embodiment of the 19 to 22 control system.

Including the ninth embodiment is configured by using the present embodiment.

Also in this embodiment, like the embodiment of 33 to 41 to treat the front end side of the image of the entire image or a wide range of the pipe as a mark imaging unit, even if the position detection of the mark good. In that case, usually, an electromagnetic wave distance measurement can just measure the distance between the points, that is, the distance between the line and the surface can not be measured, it is preferable that the mark measuring part the tip side of the head or pipe.

### EXAMPLE 49

Movement control unit of the mobile apparatus, by adjusting the magnitude and direction of the magnetic force exerted on the head by the magnetic field of the electromagnet is granted, the pipe of the head as plugged substantially straight with respect to the target position 48 and the position control system according to an embodiment of the movement control unit capable of performing the traveling direction control.

This embodiment, in the 48th embodiment, is obtained by limiting the operation of the movement control part of the main movement apparatus.

### EXAMPLE 50

The distal end side position of the head or the pipe, said head and the said pipe to form a separate mark for the imaging unit, the position control system according to an embodiment of the 48 or 49.

This embodiment, in the embodiment of the 48 or 49 is obtained mainly limit the formation position of the mark for the imaging unit.

### EXAMPLE 51

The material of the head is capable of transmitting captured material by the fluoroscopic imaging sensor, the position control system according to an embodiment of the 48 or 49.

This embodiment, in the embodiment of the 48 or 49 is obtained mainly limit the formation position of the mark for the imaging unit.

### EXAMPLE 52

The distal end side position of the head or the pipe, said head and the said pipe to form a mark separate from the measurement unit, the position control system according to an embodiment of the 48 or 49.

This embodiment, in the embodiment of the 48 or 49 is obtained mainly limit the formation position of the mark for the measurement unit.

### EXAMPLE 53

The material of the head is a substance capable of transmitting the ranging by the transmission distance measuring sensor, the position control system according to an embodiment of the 48 or 49.

This embodiment, in the embodiment of the 48 or 49 is obtained mainly limit the formation position of the mark for the measurement unit.

### EXAMPLE 54

Position detecting section of the position detection apparatus has an image recognition processing unit capable of performing an image recognition process in the position detecting unit capable of detecting a position of the head in the procedure below 1-5 There, the position control system according to any one of the embodiments of the 48 to 53.
1. Prepare the image data by the imaging unit.
2. The in the image data to locate the portion corresponding to the mark for the imaging unit by the image recognition processing unit to determine the position of the mark for the imaging unit in the imaging range of the image data.
3. In advance, by the transmission distance measurement sensor, it is better that be measuring the distance between the measuring unit for the mark and the transmission distance measurement sensor.

In addition, in advance, we are better that be measuring the three-dimensional position of the three-dimensional position and the fluoroscopic distance measurement sensor of the fluoroscopic imaging sensor.

By Pythagorean theorem, the distance of transmission and distance measurement sensor and the mark for the measurement unit, the position of the mark for the imaging unit in the shooting range of the image data, three-dimensional position of the fluoroscopic imaging sensor, the transmission distance measurement Using three-dimensional position of use sensors to determine the distance between the position where the position between the imaging unit marks on the fluoroscopic imaging sensor mark the imaging unit is reflected.
4. The distance between the position where the position between the imaging unit marks on the fluoroscopic imaging sensor mark the imaging unit is reflected to the depth coordinates of the position of the mark for the imaging unit in the imaging range of the image data, the image We ask the three-dimensional position of the mark for the imaging unit in the shooting range of the data.
5. The three-dimensional position of the mark for the imaging unit in the imaging range of the image data, in addition to three-dimensional position of the fluoroscopic imaging sensor, and the position of the head.

This embodiment, in the embodiment of the 48th to 53, is obtained by primarily limiting the procedure for position detection of the head.

This embodiment includes a three-dimensional position of the fluoroscopic imaging sensor having a photographing unit, also the embodiment in which it is better that be measured by such pre-human hands, such as during manufacture of the imaging unit. For example, when performing imaging by using a giant CCD sensor, usually, it does not move by securing a large CCD sensor in position, and corresponds to the present embodiment.

Further, this embodiment, the three-dimensional position of the perspective distance measurement sensor having a measuring unit, also the embodiment in which it is better that be measured by such pre-human hands, such as during manufacture of the measuring unit contains. For example, when performing a measurement using an electromagnetic wave distance measuring sensor, usually, it does not move by an electromagnetic wave distance measuring sensor fixed to a predetermined position, and corresponds to the present embodiment.

### EXAMPLE 55

Position detecting section of the position detection apparatus has an image recognition processing unit capable of performing an image recognition process in the position detecting unit capable of detecting a position of the head in the following 1-4 step There, the position control system according to any one of the embodiments of the 48 to 53.
1. Prepare the image data by the imaging unit.
2. The in the image data to locate the portion corresponding to the mark for the imaging unit by the image recognition processing unit to determine the position of the mark for the imaging unit in the imaging range of the image data.
3. In advance, by the transmission distance measurement sensor, it is better that be measuring the distance between the measuring unit for the mark and the transmission distance measurement sensor.
   In addition, in advance, we are better that be measuring the three-dimensional position of the three-dimensional position and the fluoroscopic distance measurement sensor of the fluoroscopic imaging sensor.
   By Pythagorean theorem, the distance of transmission and distance measurement sensor and the mark for the measurement unit, the position of the mark for the imaging unit in the shooting range of the image data, three-dimensional position of the fluoroscopic imaging sensor, the transmission distance measurement using the three-dimensional position of use sensors to determine the difference between the three-dimensional position of the fluoroscopic distance measurement sensor and the mark for the measurement unit.
4. The difference between the three-dimensional position, in addition to three-dimensional position of the fluoroscopic distance measurement sensor, and the position of the head.

This embodiment, in the embodiment of the 48th to 53, is obtained by primarily limiting the procedure for position detection of the head.

This embodiment includes a three-dimensional position of the fluoroscopic imaging sensor having a photographing unit, also the embodiment in which it is better that be measured by such pre-human hands, such as during manufacture of the imaging unit. For example, when performing imaging by using a giant CCD sensor, usually, it does not move by securing a large CCD sensor in position, and corresponds to the present embodiment.

Further, this embodiment, the three-dimensional position of the perspective distance measurement sensor having a measuring unit, also the embodiment in which it is better that be measured by such pre-human hands, such as during manufacture of the measuring unit contains. For example, when performing a measurement using an electromagnetic wave distance measuring sensor, usually, it does not move by an electromagnetic wave distance measuring sensor fixed to a predetermined position, and corresponds to the present embodiment.

### EXAMPLE 56

Perspective the position detection apparatus, the imaging unit is a three-dimensional position of the fluoroscopic imaging sensor having a measurable fluoroscopic imaging sensor position detecting section having, by the fluoroscopic imaging sensor position detecting unit, with said imaging unit and a position detector capable of measuring the three-dimensional position of the imaging sensor, the position control system according to an embodiment of the 54 or 55.

This embodiment, in the embodiment of the 54 or 55, the three-dimensional position of the fluoroscopic imaging sensor having a photographing unit, rather than measures such as the pre-human hand, when it is measured by a dedicated position sensor It is the embodiment of such.

### EXAMPLE 57

The position detection apparatus, the three-dimensional position of the transmission distance measurement sensor has a measurement possible transmission distance measuring sensor position detection unit, by the transmission distance measuring sensor position detection unit, of the transmission distance measuring sensor and a position detector capable of measuring the three-dimensional positions, the position control system according to the embodiment of the 54th to 56.

This embodiment, in the embodiment of the 54th to 56, the three-dimensional position of the perspective distance measurement sensor having a measuring unit, rather than measures such as the pre-human hand, is measured by a dedicated position sensor If it is the embodiment of such.

### EXAMPLE 58

Movement control unit of the mobile apparatus, an arm for supporting the electromagnet, and a drive unit capable of moving the arm, by adjusting the strength of the magnetic field to which the electromagnet is generated and the electromagnet by the magnetic field to impart it is possible to control the magnitude of the magnetic force acting on the head, by adjusting the position and orientation of the electromagnet, the electromagnet to control the orientation of the magnetic force exerted on the head by a magnetic field to impart The position control system according to any one of the embodiments of the 19 to 57 is a mobile control unit that can.

This embodiment, in the embodiment of the 19 to 57, is obtained by primarily limit the method of the movement control of the head.

The 1-3 and the embodiment of the fifth to 10 are constructed by using the present embodiment.

### EXAMPLE 59

Driver included in the movement control part of the mobile apparatus has a motor or a piezoelectric element having a movement unit, to the 58th embodiment of the motor unit or the piezoelectric element is a driver that is connected to the arm Position control system according.

This embodiment, in the 58th embodiment, is obtained by limiting the drive unit included in the movement control part of the main movement apparatus.

### EXAMPLE 60

The electromagnet is a plurality of electromagnets, the movement control unit of the mobile apparatus, by adjusting the magnitude of the magnetic field to each of said electromagnets to generate the magnitude of the resultant force of the magnetic force of the electromagnet acts on the head by a magnetic field to impart and a controllable movement control unit and the orientation, the position control system according to any one of the embodiments of the 19 to 57.

This embodiment, in the embodiment of the 19 to 57, is obtained by primarily limit the method of the movement control of the head.

Such fourth embodiment is configured by using the present embodiment.

### EXAMPLE 61

The pipe position control system according to any one of the embodiments of the 19 to 60 formed of a nonmagnetic material.

This embodiment, in the embodiment of the 19 to 60, is obtained by primarily limit the material of the pipe.

### EXAMPLE 62

The head position control system according to any one of the embodiments of the 19 to 61 in which the tip is dissected capable to form a living tissue.

This embodiment, in the embodiment of the 19 to 61 is obtained mainly limit the shape of the head tip.

### EXAMPLE 63

The head includes a first head portion having a narrowed shape toward the tip, are arranged said first head section by a predetermined distance, the second having a narrowed shape toward the rear end a head portion, said die including first and a second connecting portion connecting the head portion, the pipe 19 of the tip is provided so as to be positioned between said first and second head portions The position control system according to any one of the embodiments of to 62.

This embodiment, in the embodiment of the 19 to 62, is obtained by primarily limit the overall shape of the head.

### EXAMPLE 64

The pipe position control system according to any one of the embodiments of the 19 to 63 in which the tip is dissected capable to form a living tissue.

This embodiment, in the embodiment of the 19 to 63 is obtained mainly limit the shape of the pipe tip.

### EXAMPLE 65

The maximum width of the head is at 100µm or less, the magnetic field moving the controller of the mobile apparatus, which has an arm for supporting the electromagnet, and a drive unit capable of moving the arm, the electromagnets generate Adjust the strength, the magnetic field the electromagnet grants it is possible to control the magnitude of the magnetic force acting on the head, the magnetic field by adjusting the position and orientation of the electromagnet, the electromagnet grant by is a movement control unit capable of controlling the direction of the magnetic force acting on the head, drive unit with a movement control part of the mobile apparatus has a motor or a piezoelectric element having a movement unit, said movement unit or the piezoelectric element is a drive unit connected to said arm, the position detection apparatus includes a photographing portion capable of photographing the mark by the fluoroscopic imaging sensor includes a fluoroscopic imaging sensor is captured by the imaging unit the above is a position detection apparatus having a detectable position detector the position of the head based on the mark, the distal end side position of the head or the pipe, to form separately the mark from said head and said pipe to the fluoroscopic imaging sensor imaging unit having an image sensor pair of two-dimensional, the position detection apparatus, the imaging unit three-dimensional position of the fluoroscopic imaging sensor having a measurable fluoroscopic imaging sensor position detection a unit, by the fluoroscopic imaging sensor position detector is capable of measuring the position detector the three-dimensional position of the fluoroscopic imaging sensor with said imaging unit, the position detecting section of the position detection apparatus, an image recognition which has an image recognition processing unit capable of performing the process, a position detector capable of detecting the position of the head in the following 1-3 steps to the quote nineteenth embodiment 23 position control system according to an embodiment of the.
1. Prepare the image data by the imaging unit. The image data is a pair of image data taken from a direction perpendicular to each other.
2. The portion corresponding to the mark in each of the image data by searching through the image recognition processing unit obtains a two-dimensional position of the mark in the imaging range of the image data, then the in the imaging range of the pair of the image data by combining the two-dimensional position of the mark in the orthogonal direction to obtain the three-dimensional position of the mark in the imaging range of the image data.
3. Previously said it is also possible to measure the three-dimensional position of the fluoroscopic imaging sensor imaging unit has, on the three-dimensional position of the mark in the imaging range of the image data, and adding the three-dimensional position of the fluoroscopic imaging sensor with said imaging unit Te, and the position of the head. The present embodiment, the first 19, second 20, third 23, 24th, 25th, 26th, 28th, 29th, 30th, combining the technical features of the first 58, second 59 embodiment described was it is intended.

### EXAMPLE 66

The movement apparatus according to any one of the embodiments of the first to 65.

This embodiment is a mobile apparatus according to any one of the embodiments of the first to 65.

The purpose of this embodiment, with respect to the body of a target position, the cells were injected liquid such as anti-cancer agents without destruction as possible, injection-aspiration system capable of aspirating a liquid, such as cytosol. The embodiment of the present invention is to provide a mobile apparatus to configure.

According to this embodiment, with respect to the body of a target position, the cells were injected liquid such as anti-cancer agents without destruction as possible, injection-aspiration system capable of aspirating a liquid, such as cytosol and it can be constructed.

### EXAMPLE 67

The position detection apparatus according to any one of the embodiments of the first to 65.

This embodiment is a position detection apparatus according to any one of the embodiments of the first to 65.

The purpose of this embodiment, with respect to the body of a target position, the cells were injected liquid such as anti-cancer agents without destruction as possible, injection-aspiration system capable of aspirating a liquid, such as cytosol the embodiment of the present invention is to provide a position detection apparatus for configuration.

According to this embodiment, with respect to the body of a target position, the cells were injected liquid such as anti-cancer agents without destruction as possible, injection-aspiration system capable of aspirating a liquid, such as cytosol and it can be constructed.

An embodiment of the present invention is not limited to the embodiments described above, and the embodiments of the present invention can be modified and implemented in various ways to the extent that the gist of the embodiments of the present invention is not changed.

For example, in also the other embodiment than the EXAMPLE 10, a combination of an imaging unit of a position detection apparatus and a mark such as it is bright darkly at just a position of the mark in an image data which is prepared using the imaging unit of the position detection apparatus may be used instead of a combination of an imaging unit of the position detection apparatus and a mark such as just a position of the mark is photographed darkly in an image data which is prepared using the imaging unit of the position detection apparatus clearly like the EXAMPLE 10. In other words, photographing using an imaging unit of a position detection apparatus may be negative type instead of positive type.

### INDUSTRIAL APPLICABILITY

Position control system of the embodiment of the present invention is industrially useful because it can constitute an injection-suction system capable of injecting liquid such as anti-cancer agents to a target position of a body and sucking a liquid such as cytosol from a target position of a body without destruction as possible.

### REFERENCE SIGNS LIST

10:injection-suction system, 20:fine head, 30:ultra-fine pipe, 30a:inclined surface, 40:movement apparatus, 50:position detection apparatus, 70:mold, 100:injection-suction apparatus, 200:first head portion, 200a ∼ 200c:sides, 200d:bottom surface, 210second head portion, 210a:bottom surface, 210b ∼ 210e:sides, 210f:top surface, 220:connecting portions, 230:head portion, 230a, 230b:inclined surfaces, 400:electromagnet, 410A:first arm, 410B:second arm, 420A:first rotary drive unit, 420B:second rotary drive unit, 420C:third rotary drive unit, 430:base, 440:control unit, 450:operation unit, 460-467:electromagnets, 468:control unit, 469:operation unit, 500:X-ray CCD sensor(s), 510A:first arm(s), 510B:second arm(s), 520A:first rotary drive unit(s), 520B:second rotary drive unit(s), 520C:third rotary drive unit(s), 530:base(s), 540:control unit, 550:operating unit, 560:display unit, 570:MRI sensor, 571:slide drive unit, 572:base, 573:control unit, 574:operation unit, 575:display unit, 580:CT sensor, 581:slide drive unit, 582:base, 583:control unit, 584:operation unit, 585:display unit, 590:ultrasonic inspection probe, 591A:first arm, 591B:second arm, 592:extension/contraction drive unit, 593:slide drive unit, 594:base, 595:control unit, 596:operation unit, 597:display unit, 598:piezoelectric element, 600-602:radio rangefinders, 603:control unit, 604:display unit, 610:radio rangefinder, 611:control unit, 612:operation unit, 613:display unit, 620:irradiation units, 621:control unit, 700, 710:space portions, 720:through-hole, 730: recesses, 1000:cancel cells, 1100:group of cancer cells, 1200:anti-cancer agent, 1300:summarized one group of cancer cells, 2000:film, 2000a:groove, 2010:film, P:human body, Q:virtual cube

## Claims

1. A position control system comprising:
a movement apparatus (40), including an electromagnet (400;460 ∼ 67) capable of applying a magnetic field to a head (20) that is formed from a magnetic material and can be moved through a body (P) by the magnetic field, and a movement control unit (440;468) capable of controlling a magnitude and an orientation of a magnetic force that acts on the head (20) in response to the magnetic field applied by the electromagnet (400;460 ∼ 467); and
a position detection apparatus (50) being capable of determining a position of a mark existing on the head (20) or in a tip end side position of a pipe (30) which is attached to the head (20) with a tip end thereof open and through which a liquid can be injected or suctioned via an opening portion in the tip end, the position detection apparatus (50) being capable of detecting a position of the head (20) on the basis of the position of the mark,
the movement control unit (440;468) of the movement apparatus (40) is capable of controlling an advancement direction of the head (20) by adjusting the magnitude and the orientation of the magnetic force that acts on the head (20) in response to the magnetic field applied by the electromagnet (400;460 ∼ 467) so that the pipe (30) is inserted substantially linearly into a target position.

2. The position control system according to claim 1, **characterized in that** a maximum width of the head (20) is no greater than 100 micrometer.

3. The position control system according to claim 1 or 2, **characterized in that** the position detection apparatus (50) includes:
an imaging unit having a radiographic imaging sensor (500;570;580;590) and capable of capturing an image of the mark using the radiographic imaging sensor (500;570;580;590); and
a position detection unit (540;573;583;595;603;611;621) capable of detecting the position of the head (20) on the basis of the image of the mark captured by the imaging unit.

4. The position control system according to claim 3, **characterized in that** the position detection unit (540;573;583;595;603;611;621) of the position detection apparatus (50) includes an image recognition processing unit capable of performing image recognition processing, and is capable of detecting the position of the head (20) by implementing following procedures 1 to 3:
1. preparing image data using the imaging unit;
2. determining the position of the mark within an imaging range of the image data by searching the image data for a part corresponding to the mark using the image recognition processing unit; and
3. measuring, in advance, a three-dimensional position of the radiographic imaging sensor (500;570;580;590) provided in the imaging unit, and determining the position of the head (20) by adding the three-dimensional position of the radiographic imaging sensor (500;570;580;590) provided in the imaging unit to the position of the mark within the imaging range of the image data.

5. The position control system according to claim 1 or 2, **characterized in that** the position detection apparatus (50) includes:
a measurement unit that has at least three transmission distance measurement sensors (600 ∼ 602;610) capable of measuring a distance to the mark using a transmission method, and that can measure respective distances between the transmission distance measurement sensors (600 ∼ 602;610) and the mark using the transmission distance measurement sensors (600 ∼ 602;610); and
a position detection unit (540;573;583;595;603;611;621) capable of detecting the position of the head (20) on the basis of the distances.

6. The position control system according to claim 5, **characterized in that** the position detection unit (540;573;583;595;603;611;621) of the position detection apparatus (50) is capable of detecting the position of the head (20) by implementing following procedures 1 to 3:
1. preparing the respective distances between the transmission distance measurement sensors (600 ∼ 602;610) and the mark using the transmission distance measurement sensors (600 ∼ 602;610);
2. measuring three-dimensional positions of the transmission distance measurement sensors (600 ∼ 602;610) in advance, and determining a difference in three-dimensional position between at least one of the transmission distance measurement sensors (600 ∼ 602;610) and the mark by Pythagoras' theorem using the distances and the three-dimensional positions of the transmission distance measurement sensors (600 ∼ 602;610); and
3. obtaining the position of the head (20) by adding the three-dimensional positions of the transmission distance measurement sensors (600 ∼ 602;610) to the difference determined in 2.

7. The position control system according to claim 1 or 2, **characterized in that** the mark is constituted by an imaging unit mark and a measurement unit mark, and
the position detection apparatus (50) includes:
an imaging unit having a radiographic imaging sensor (500;570;580;590) and capable of capturing an image of the imaging unit mark using the radiographic imaging sensor (500;570;580;590);
a measurement unit that has a transmission distance measurement sensor (600 ∼ 602;610) capable of measuring a distance to the measurement unit mark using a transmission method, and that can measure a distance between the transmission distance measurement sensor (600 ∼ 602;610) and the measurement unit mark using the transmission distance measurement sensor (600 ∼ 602;610); and
a position detection unit (540;573;583;595;603;611;621) capable of detecting the position of the head (20) on the basis of the image of the imaging unit mark, captured by the imaging unit, and the distance.

8. The position control system according to claim 7, **characterized in that** the position detection unit (540;573;583;595;603;611;621) of the position detection apparatus (50) includes an image recognition processing unit capable of performing image recognition processing, and is capable of detecting the position of the head (20) by implementing following procedures 1 to 5:
1. preparing image data using the imaging unit;
2. determining a position of the imaging unit mark within an imaging range of the image data by searching the image data for a part corresponding to the imaging unit mark using the image recognition processing unit;
3. measuring, in advance, a distance between the transmission distance measurement sensor (600 ∼ 602;610) and the measurement unit mark using the transmission distance measurement sensor (600 ∼ 602;610),
measuring, in advance, a three-dimensional position of the radiographic imaging sensor (500;570;580;590) and a three-dimensional position of the transmission distance measurement sensor (600 ∼ 602;610), and
determining a distance between the position of the imaging unit mark and a position in which the imaging unit mark is projected on the radiographic imaging sensor (500;570;580;590) by Pythagoras' theorem using the distance between the transmission distance measurement sensor (600 ∼ 602;610) and the measurement unit mark, the position of the imaging unit mark within the imaging range of the image data, the three-dimensional position of the radiographic imaging sensor (500;570;580;590), and the three-dimensional position of the transmission distance measurement sensor (600 ∼ 602;610);
4. determining a three-dimensional position of the imaging unit mark within the imaging range of the image data by setting the distance between the position of the imaging unit mark and the position in which the imaging unit mark is projected on the radiographic imaging sensor (500;570;580;590) as a depth coordinate of the position of the imaging unit mark within the imaging range of the image data; and
5. obtaining the position of the head (20) by adding the three-dimensional position of the radiographic imaging sensor (500;570;580;590) to the three-dimensional position of the imaging unit mark within the imaging range of the image data.

9. The position control system according to claim 7, **characterized in that** the position detection unit (540;573;583;595;603;611 ;621) of the position detection apparatus (50) includes an image recognition processing unit capable of performing image recognition processing, and is capable of detecting the position of the head (20) by implementing following procedures 1 to 4:
1. preparing image data using the imaging unit;
2. determining a position of the imaging unit mark within an imaging range of the image data by searching the image data for a part corresponding to the imaging unit mark using the image recognition processing unit;
3. measuring, in advance, a distance between the transmission distance measurement sensor (600 ∼ 602;610) and the measurement unit mark using the transmission distance measurement sensor (600 ∼ 602;610),
measuring, in advance, a three-dimensional position of the radiographic imaging sensor (500;570;580;590) and a three-dimensional position of the transmission distance measurement sensor (600 ∼ 602;610), and
determining a difference in three-dimensional position between the measurement unit mark and the transmission distance measurement sensor (600 ∼ 602;610) by Pythagoras' theorem using the distance between the transmission distance measurement sensor (600 ∼ 602;610) and the measurement unit mark, the position of the imaging unit mark within the imaging range of the image data, the three-dimensional position of the radiographic imaging sensor (500;570;580;590), and the three-dimensional position of the transmission distance measurement sensor (600 ∼ 602;610); and
4. obtaining the position of the head (20) by adding the three-dimensional position of the transmission distance measurement sensor (600 ∼ 602;610) to the difference in three-dimensional position.

10. The position control system according to any one of claims 1 to 9, **characterized in that** the movement control unit (440;468) of the movement apparatus (40) includes an arm (410A, 410B) that supports the electromagnet (400;460 ∼ 467) and a drive unit (420A, 420B, 420C) capable of moving the arm (410A, 410B), and is capable of controlling the magnitude of the magnetic force that acts on the head (20) in response to the magnetic field applied by the electromagnet (400;460 ∼ 467) by adjusting a strength of the magnetic field generated by the electromagnet (400;460 ∼ 467), and of controlling the orientation of the magnetic force that acts on the head (20) in response to the magnetic field applied by the electromagnet (400;460 ∼ 467) by adjusting a position and an orientation of the electromagnet (400;460 ∼ 467).

11. The position control system according to any one of claims 1 to 9, **characterized in that** the electromagnet (400;460 ∼ 467) is constituted by a plurality of electromagnets (460 ∼ 467), and
the movement control unit (440;468) of the movement apparatus (40) is capable of controlling a magnitude and an orientation of a resultant force of magnetic forces that act on the head (20) in response to magnetic fields applied respectively by the electromagnets by adjusting strengths of the magnetic fields generated respectively by the electromagnets.

12. The position control system according to any one of claims 1 to 11, **characterized in that** the pipe (30) is formed from a non-magnetic material.

13. The position control system according to any one of claims 1 to 12, **characterized in that** the head (20) is formed such that a tip end thereof is capable of making incisions in living tissue.

14. The position control system according to any one of claims 1 to 13, **characterized in that** the head (20) includes:
a first head portion (200) shaped to taper toward a tip end thereof;
a second head portion (210) disposed at a predetermined distance from the first head portion (200) and shaped to taper toward a rear end thereof and
a connecting portion (220) that connects the first and second head portions (210), and
the pipe (30) is provided such that the tip end thereof is positioned between the first and second head portions (200, 210).

15. The position control system according to any one of claims 1 to 14, **characterized in that** the pipe (30) is formed such that the tip end thereof is capable of making incisions in living tissue.
